# EUROPEAN PATENT SPECIFICATION

(11) **EP 3 325 514 B1**
(45) Date of publication and mention of the grant of the patent: **25.09.2019**
(21) Application number: 16741919.1
(22) Date of filing: 19.07.2016
(51) Int. Cl.: C07K 16/32, C07K 14/47, C12N 15/62

(54) **HER2 BINDING PROTEINS BASED ON DI-UBIQUITIN MUTEINS**
HER2 BINDING PROTEINS BASED ON DI-UBIQUITIN MUTEINS
PROTÉINES DE LIAISON À HER2 À BASE DE DI-UBIQUITINE MUTÉINES

(30) Priority: 20.07.2015 EP 15177548
(43) Date of publication of application: 30.05.2018
(73) Proprietor: NAVIGO PROTEINS GMBH, 06120 Halle/Saale (DE)
(72) Inventor: SETTELE, Florian, 06120 Halle/Saale (DE); ZWARG, Madlen, 06120 Halle/Saale (DE); GLOSER, Manja, 06120 Halle/Saale (DE); BOSSE-DOENECKE, Eva, 06120 Halle/Saale (DE); FIEDLER, Erik, 06120 Halle/Saale (DE); HAUPTS, Ulrich, 06120 Halle/Saale (DE)
(74) Representative: Grünecker Patent- und Rechtsanwälte PartG mbB
(86) International application number: PCT/EP2016/067207
(87) International publication number: WO 2017/013129

(56) References cited:
- EP-A1- 2 738 180
- WO-A1-2014/094799

## Description

### Field of the invention

The present invention relates to new Her2 binding molecules based on di-ubiquitin muteins. The invention further refers to Her2 binding proteins optionally fused or conjugated to a moiety modulating pharmacokinetics or to a therapeutically or diagnostically active component. The invention further relates to the use of these Her2 binding proteins in medicine, preferably for use in the diagnosis or treatment of cancer.

### Background of the invention

Increased expression of the membrane-bound receptor tyrosine kinase Her2 plays an important role in the development and progression of many breast carcinomas, but also in ovarian, stomach, and uterine cancer, particularly with aggressive forms of cancer. Overexpression of this oncogene is reported for malignancies, predominantly in malignancies of epithelial origin, and is associated with cancer recurrence and poor prognosis.

The three domain protein (extracellular, transmembrane, intracellular tyrosine kinase domain) is mediating cell proliferation and inhibiting apoptosis. Upon binding of a ligand to the extracellular domain of Her2, Her2 forms dimers
with the receptor whereby the intracellular domain of Her2 is activated which mediates cellular processes such as proliferation, differentiation, migration, or apoptosis. Thus, modulating the function of Her2 is an important approach for the development of cancer therapeutics, in particular those based on monoclonal antibodies binding to the extracellular domain of Her2. Therapeutic anti-Her2 monoclonal antibodies such as Trastuzumab or Pertuzumab are available for treatments of cancer, in particular breast cancer.

EP 2 738 180 A1 relates to genetically engineered antibody mimetic proteins (DARPins), and describes in particular bispecific constructs with dual specificity for different domains of Her2. WO 2014/094799 A1 describes ubiquitin-derived Affilin® molecules as a means for prolonging serum half-life of pharmaceutically active compounds.

### Technical problems underlying the present invention

Monoclonal antibodies have major disadvantages such as a complex molecular structure, a large size, and challenging production methods. Furthermore, treatment of diseases with currently available Her2 binding molecules is not effective in all patients and may have severe side effects.

Needless to say that there is a strong medical need to effectively treat cancer with improved novel agents, in particular efficient tumor targeted therapeutics and diagnostics. There is an ongoing need to find alternative to current therapies and diagnosis, i.e. to substitute Her2 monoclonal antibodies by smaller and less complex Her2 specific molecules such as non-immunoglobulin based Her2 binding agents.

To overcome the disadvantages of antibodies, novel Her2 binding molecules suitable for diagnostic and therapeutic applications should include characteristics such as affinity to Her2, specificity to Her2, and high stability.

It is thus an objective of the present invention to provide novel Her2 binding non-immunoglobulin molecules for new and improved strategies in the treatment and diagnosis of cancer with Her2 overexpression. In particular, it is an objective to provide novel binding proteins which have high affinity and specificity to Her2, combined with a less complex and smaller structure, for example for enabling a simplified molecular engineering.

A solution is described herein by small Her2 binding proteins such as non-immunoglobulin based binding agents, in particular by Her2 binding molecules based on ubiquitin muteins (also known as Affilin® molecules).

Compared to antibodies, a significant advantage of the Her2 binding proteins described herein is the reduced complexity in terms of (i) reduced size (e.g. of maximal 152 amino acids), (ii) simple molecular structure (one chain compared to four chains of an antibody), and (iii) posttranslational modifications possible but not required for full functionality. The Her2 binding proteins described herein provide molecular formats with favorable physicochemical properties (such as stability and solubility), high-level expression, and allow easy production methods. The Her2 specific Affilin molecules are characterized by high affinity for Her2, by specificity for a Her2, and by high stability, and provide novel therapeutic and diagnostic possibilities.

The above-described objectives and advantages are achieved by the subject-matter of the invention, which is defined by the appended claims. The present invention meets the needs presented above by providing examples for specific Her2 binding proteins based on di-ubiquitin muteins with substitutions in at least 12 amino acid positions of di-ubiquitin. Preferred embodiments of the invention are included in the dependent claims as well as in the corresponding passages of the following description, examples and figures. The above overview does not necessarily describe all problems solved by the present invention.

### Summary of the invention

In a first aspect the present invention relates to a Her2 binding protein wherein the Her2 binding protein comprises an amino acid sequence wherein at least 12 amino acids selected from positions R42, I44, H68, V70, R72, L73, R74, K82, L84, Q138, K139, E140, S141, and T142 of di-ubiquitin having the amino acid sequence of SEQ ID NO: 4 are substituted, and wherein the Her2 binding protein has at least 85 % sequence identity to di-ubiquitin of SEQ ID NO: 4, and wherein the Her2 binding protein has a binding affinity (K_{D}) of less than 700 nM for Her2, wherein the amino acid at
position 42 is substituted by a polar amino acid,
position 44 is substituted by a hydrophobic or polar amino acid,
position 68 is substituted by an aromatic amino acid,
position 70 is substituted by an aromatic amino acid,
position 72 is substituted by a polar or aromatic amino acid,
position 73 is substituted by any amino acid but not basic or acidic amino acid,
position 74 is substituted by an aromatic, basic or polar amino acid,
position 82 is substituted by any amino acid but not basic or acidic amino acid,
position 84 is substituted by a basic or acidic amino acid,
position 138 is substituted by a basic or acidic or polar amino acid,
position 139 is substituted by an acidic or hydrophobic amino acid or glycine,
position 140 is substituted by an aromatic amino acid,
position 141 is substituted by a hydrophobic or polar or basic amino acid, and/or
position 142 is substituted by a hydrophobic or polar amino acid.

Preferably, the binding affinity determined by ELISA or by surface plasmon resonance assays.

In one embodiment, a Her2 binding protein of the present invention further comprises at least one additional molecule, preferably selected from at least one member of the groups (i), (ii) and (iii) consisting of (i) a moiety modulating pharmacokinetic behavior selected for example from a polyethylene glycol, a human serum albumin (HSA), a human serum albumin binding protein, an albumin-binding peptide, or an immunoglobulin or immunoglobulin fragments, a polysaccharide, and, (ii) a therapeutically active component, optionally selected for example from a monoclonal antibody or a fragment thereof, a cytokine, a chemokine, a cytotoxic compound, an enzyme, or derivatives thereof, or a radionuclide, and (iii) a diagnostic component, optionally selected for example from a fluorescent compound, a photosensitizer, a tag, an enzyme, or a radionuclide.

The present invention also provides, in further aspects, a nucleic acid or nucleic acids encoding the Her2 binding proteins comprising or consisting of a binding protein of the present invention, as well as a vector or vectors comprising said nucleic acid or nucleic acids, and a host cell or host cells comprising said vector or vectors.

Another aspect relates to said Her2 binding protein of the present invention for use in diagnostics or medicine, preferably for use in the diagnosis or treatment of cancer, or a nucleic acid molecule encoding said Her2 binding protein, or a vector comprising said Her2 binding protein, or a host cell comprising said Her2 binding protein, or a non-human host comprising said Her2 binding protein.

Another aspect relates to a composition comprising the Her2 binding protein of the invention, the nucleic acid molecule of the invention, the vector of the invention, or the host cell of the invention, preferably for use in the diagnosis or treatment of cancer.

Another aspect of the present invention relates to a method for the production of a Her2 binding protein of any of the preceding aspects and embodiments of the invention comprising culturing of host cells under suitable conditions and optionally isolation of the Her2 binding protein produced.

Other embodiments of the invention will become apparent from a review of the ensuing detailed description.

### Brief description of the Figures

The Figures show:

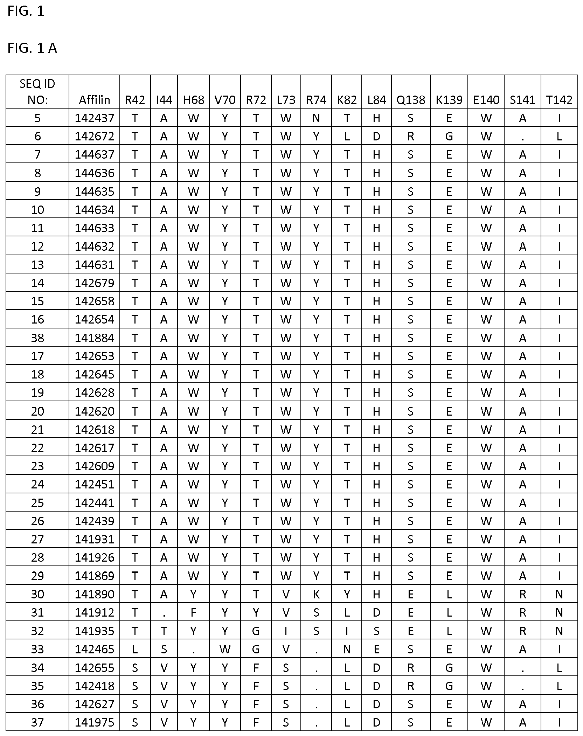

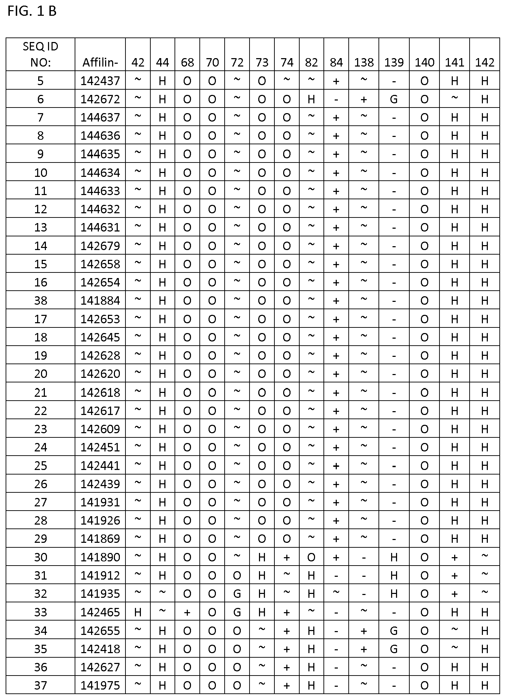

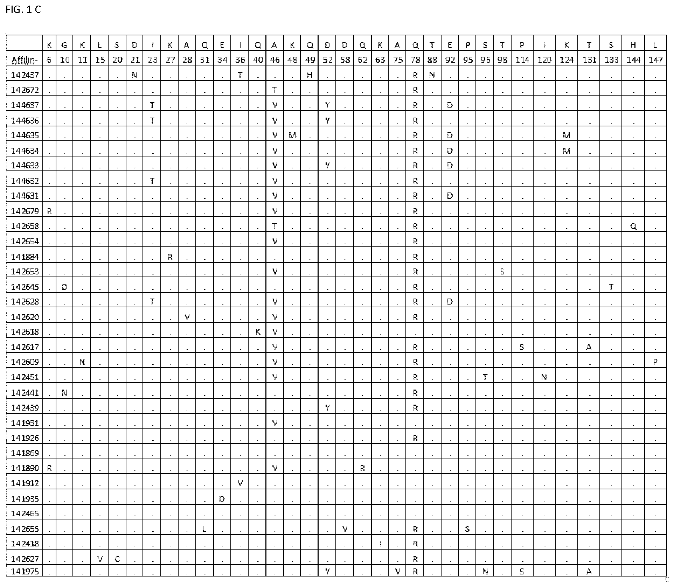

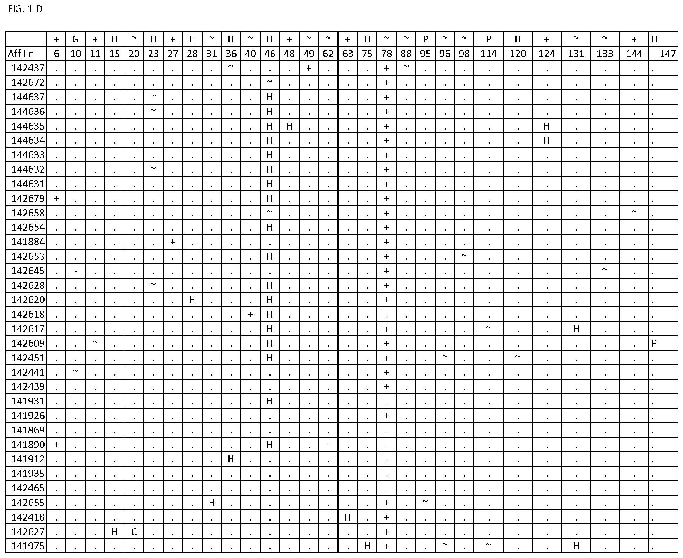

**FIG. 1** shows Her2 binding Affilin molecules.
FIG. 1 A lists positions of di-ubiquitin (SEQ ID NO: 4) that are substituted in order to generate a Her2 binding protein. In the first row, the corresponding amino acid position is listed. All Her2 binding proteins (for example, SEQ ID NOs: 5-38) are substituted at least in 12 positions selected from positions R42, I44, H68, V70, R72, L73, R74, K82, L84, Q138, K139, E140, S141, and T142 of SEQ ID NO: 4. A "." In the table refers to a wild type position (unchanged); for example, as exemplified in SEQ ID Nos: 6, 31, 33, 34, 35, 36, and 37.
FIG. 1 B shows the same amino acid exchanges as FIG. 1 A, however, the exchanges are translated according to the following code which groups amino acids with similar biophysical properties. A waved line "∼" is the symbol for" polar amino acids (T, S, N, or Q) "H" is the symbol for hydrophobic amino acids (e.g. A, M, L, V, I) "o" is the symbol for aromatic amino acids (e.g. F, W, Y), "+"the symbol for basic amino acids (e.g. K, R, H), "-"the symbol for acidic amino acids (e.g. D, E), and "G" corresponds to Glycine.
FIG. 1 C lists further substitutions; all Her2 binding proteins have 0, 1, 2, 3, 4, 5, or 6 further modifications in addition to the at least 12 substitutions selected from amino acid positions R42, 144, H68, V70, R72, L73, R74, K82, L84, Q138, K139, E140, S141, and T142 of SEQ ID NO: 4.
FIG. 1 D shows the same amino acid exchanges as FIG. 1 C, however, the exchanges are translated according to the code which groups amino acids with similar biophysical properties as described in FIG. 1 B.

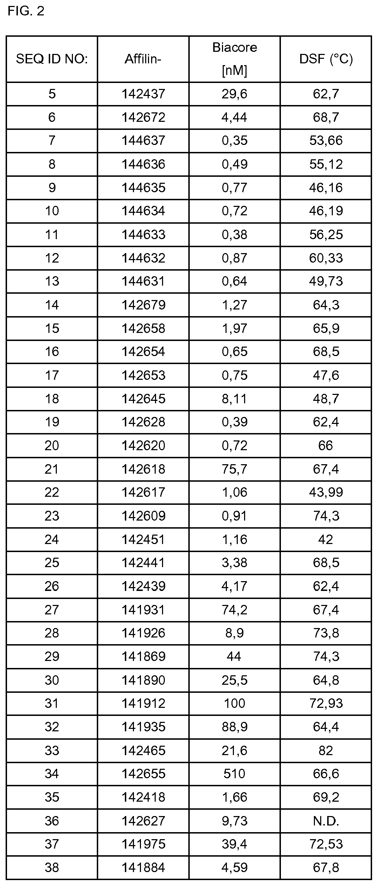

**FIG. 2****.** Biochemical characterization of Her2 binding Affilin molecules (for example, SEQ ID NOs: 5-38). Shown are binding affinities (K_{D}) as obtained from SPR assay (Biacore; third column of the table) and temperature stability (DSF; fourth column of the table).

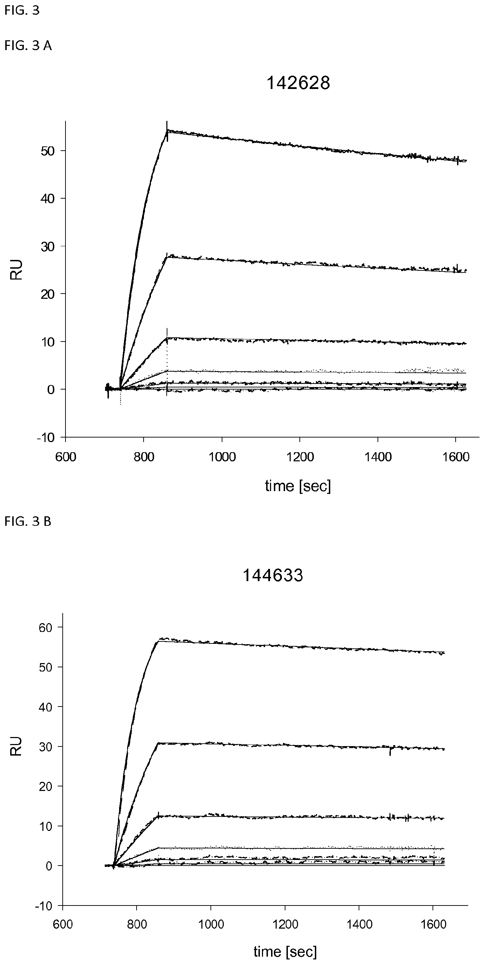

**FIG. 3****.** Analysis of Her2 binding proteins via label-free interaction assays using SPR (Biacore). Different concentrations of Affilin proteins (0, 0.137, 0.4115, 1.2345, 3.7037, 11.11, and 33.33 nM) were analyzed for binding to Her2 immobilized on a chip (Biacore) to analyze the interaction between the Affilin protein and Her2. FIG. 3 A shows the binding kinetics of Affilin-142628 to Her2. FIG. 3 B shows the binding kinetics of Affilin-144633 to Her2.

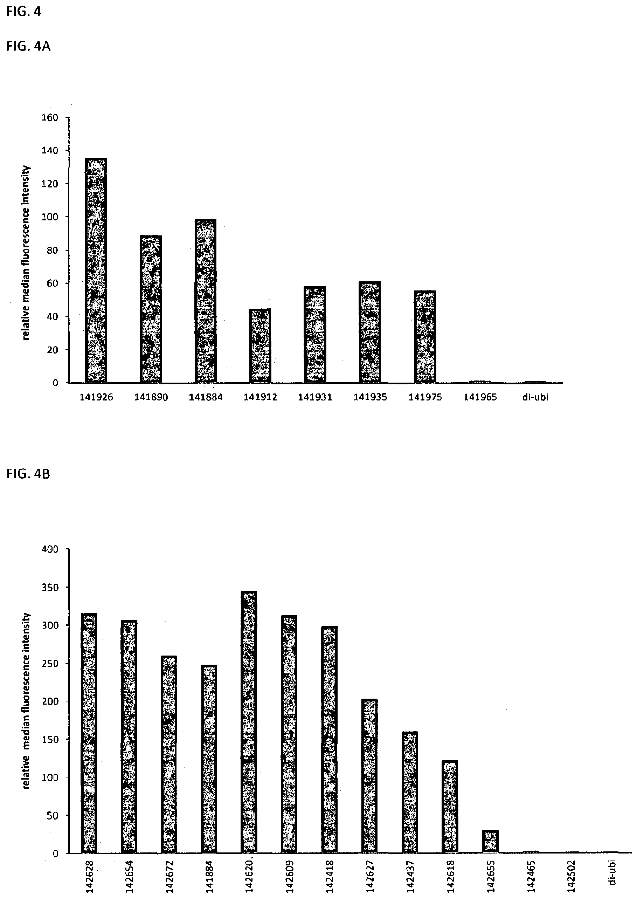

**FIG. 4****.** Functional characterization of Her2 binding proteins confirming binding to cellular Her2. The figure shows binding to exogenously Her2 expressing SkBr3 cells as determined by FACS analysis. Her2 binding proteins ("Affilin") show binding at 50 nM on SkBr3 cells (dark grey bars) and no activity on HEK/293 cells (see FIG. 4 A and FIG. 4 B). Weak or no binding to Her2 expressing SkBr3 was observed for Affilin-142655 (referred to as "142655"), Affilin-141965, Affilin-142465 (referred to as "142465"), Affilin-142502 (referred to as "142502"), and di-ubiquitin (referred to as "di-ubi").

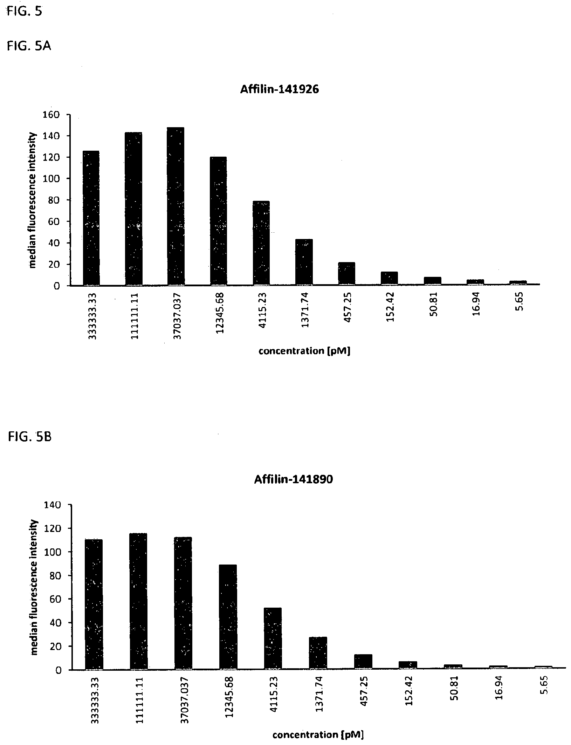

**FIG. 5****.** Concentration dependent functional binding of Her2 binding proteins to exogenously Her2 expressing SkBr3 cells as determined by flow cytometry analysis. Shown is a dilution series of 333 nM to 5.6 pM of binding protein. Affilin-141926 (FIG. 5 A) and Affilin-141890 (FIG. 5 B) show a concentration depending binding on SkBr3-cells.

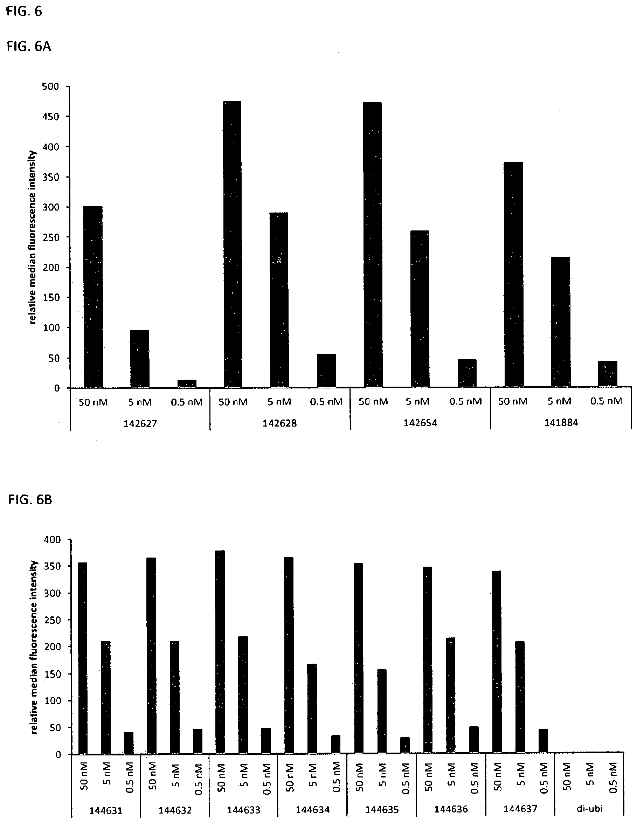

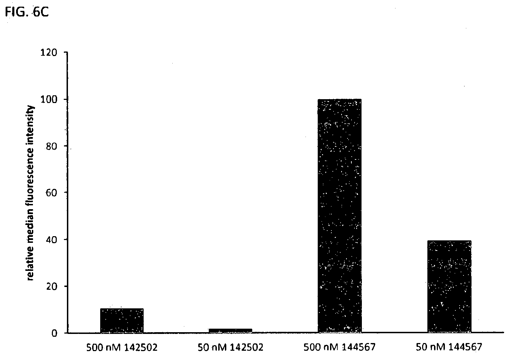

**FIG. 6****.** Functional characterization of Her2 binding proteins confirming binding to exogenously Her2 overexpressing CHO-K1 cells as determined by flow cytometry analysis. Her2 binding proteins show binding on CHO-K1-Her2 cells at concentrations of 50 nM, 5 nM, and 0.5 nM. FIG. 6 A shows the Her2-binding of Affilin-142627, Affilin-142628, Affilin-142654, and Affilin-141884; FIG. 6 B shows cellular Her 2 binding of Affilin-144631, Affilin-144632, Affilin-144633, Affilin-144634, Affilin-144635, Affilin-144636, Affilin-144637, and FIG. 6 C shows cellular Her 2 binding of Affilin-144567, and only low levels of binding of 142502 at 500 nM. Thus, cellular Her2 binding was confirmed for all binding molecules except 142502 even at the lowest concentration tested. Di-ubiquitin showed no binding on CHO-K1-Her2-cells (shown, for example, in FIG. 6 B).

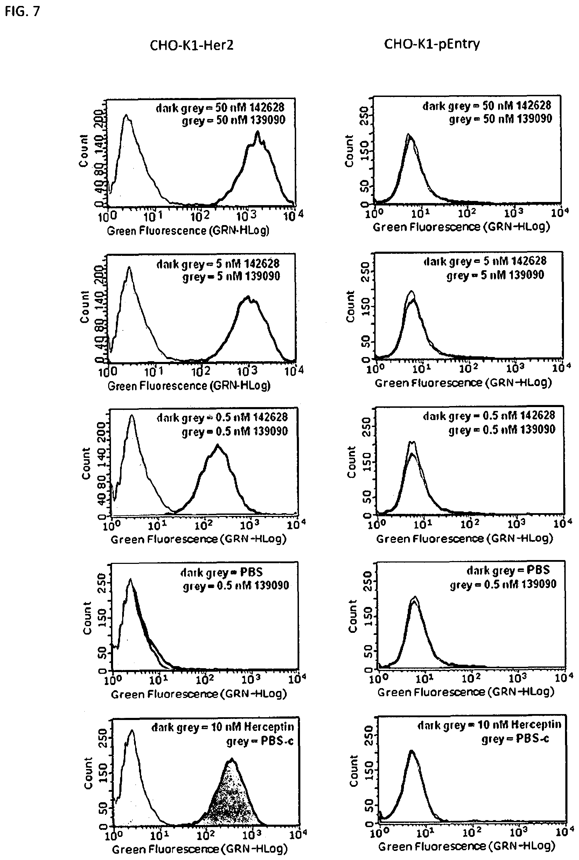

**FIG. 7****.** Concentration dependent binding of Affilin-142628. The figure shows binding of Affilin-142628 to exogenously Her2 expressing CHO-K1 cells as determined by flow cytometry (FACS analysis)(control: empty vector CHO-K1-pEntry cells). Histograms at different Affilin protein concentrations of 50 nM, 5 nM and 0.5 nM are shown in comparison to di-ubiquitin 139090 (SEQ ID NO: 4). Affilin-142628 induces a concentration depending shift on the Her2-overexpressing cell line.

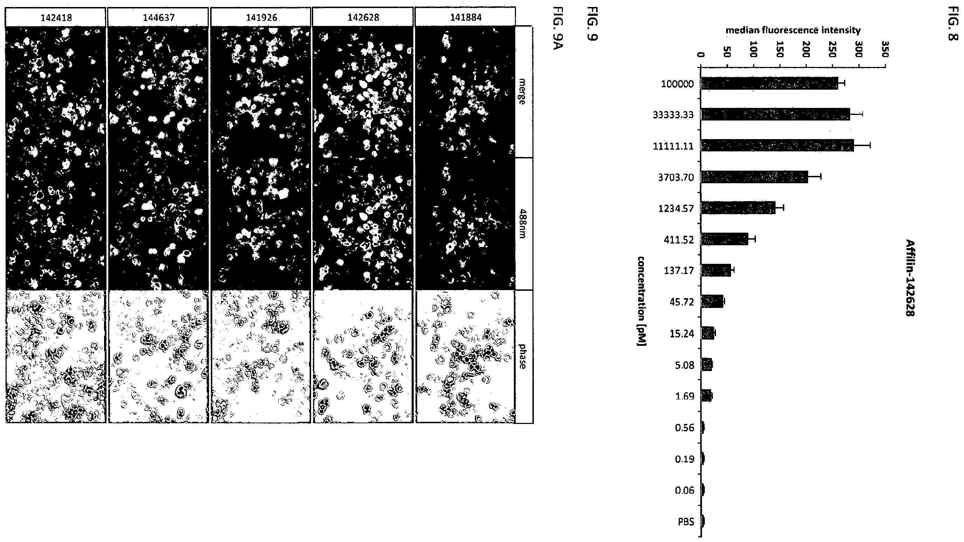

**FIG. 8****.** Concentration dependent functional binding of Her2 binding proteins to exogenously Her2 expressing SkBr3-cells as determined by flow cytometry. A dilution series of 100 nM to 0.06 pM of Affilin-142628 was used to analyze the interaction with Her2 overexpressing SkBr3 cells. FIG. 8 shows a concentration dependent binding of Affilin-142628.

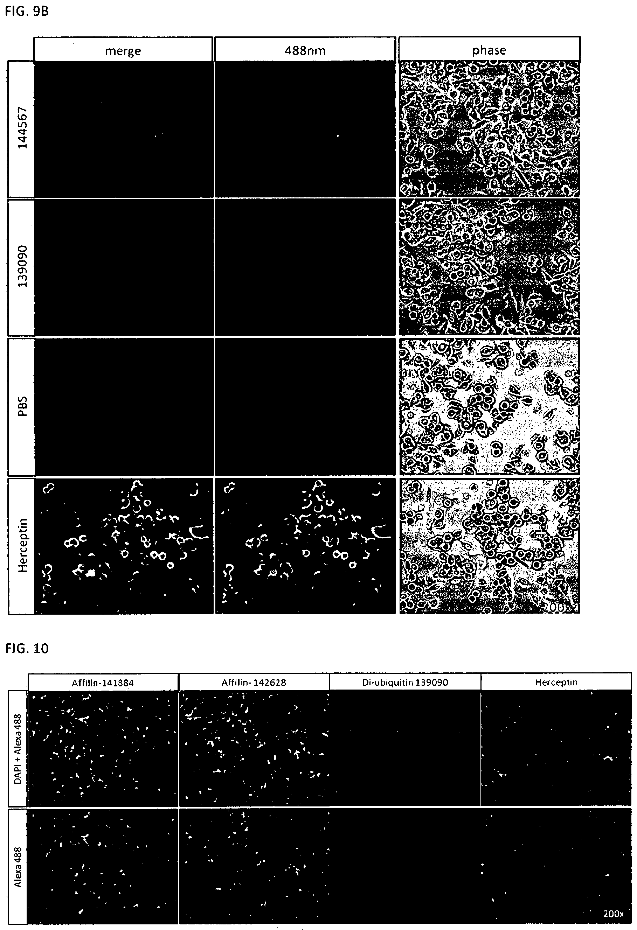

**FIG. 9** shows the binding analysis of different Her2 binding proteins on Her2-overexpressing SkBr3-cells by immunofluorescence staining. FIG. 9A shows concentrations of 50 nM Affilin-141884, Affilin-142628, Affilin-141926, Affilin-144637, Affilin-142418. FIG. 9B shows concentrations of 50 nM Affilin-144567, di-ubiquitin (139090), PBS, and Trastuzumab (Herceptin). Affilin-141884, Affilin-142628, Affilin-141926, Affilin-144637 and Affilin-142418 show a strong binding on the Her2-overexpressing cell line, whereas Affilin-144567 and di-ubiquitin (139090) do not bind to Her2 on SkBr-3 cells.
**FIG. 10** confirms that Her2 binding proteins bind to SKOV-3 xenograft tumor tissue. Shown is an immunohistological staining of 50 nM Affilin-141884 and 50 nM Affilin-142628 on Her2-expressing tumor tissue derived from human ovarian adenocarcinoma cells. Affilin-141884 and Affilin-142628 show a strong binding on Her2-expressing tissue. Di-ubiquitin (139090) shows no binding on SKOV-3 tissue slides.

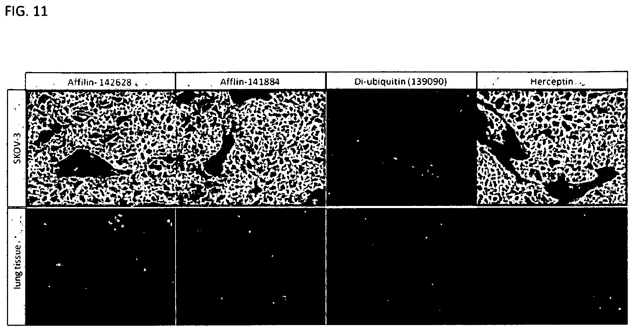

**FIG. 11** shows an immunohistological binding analysis of Her2 binding proteins on Her2-expressing SKOV-3-tumor tissue slides and lung tissue slides without Her2 expression. Affilin-141884 and Affilin-142628 show strong binding at 20 nM on SKOV-3 tissue. No binding to lung tissue was observed. In addition, no binding of Affilin-141884 and Affilin-142628 to tissue obtained from liver, heart muscle, and ovary was observed.

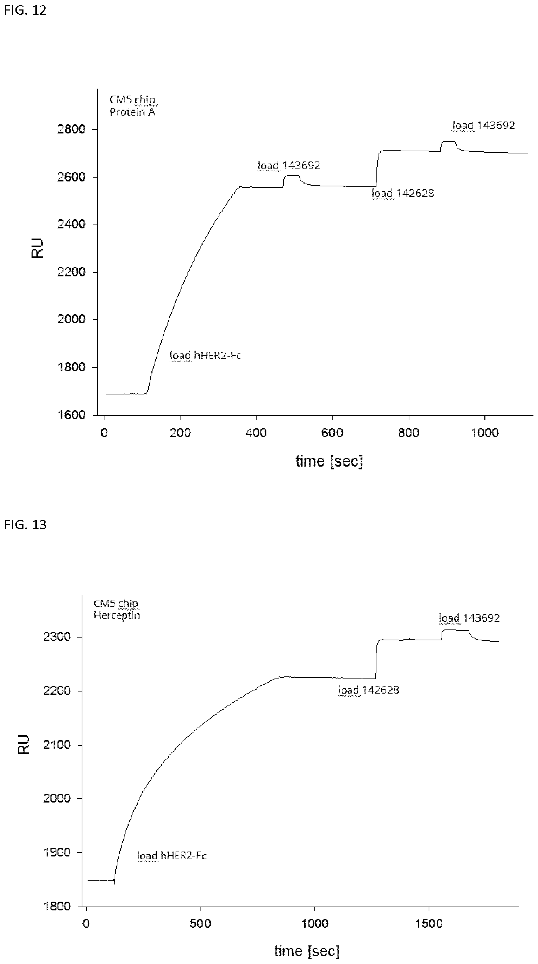

**FIG. 12** shows that Affilin-142628 and Affilin-143692 bind to different Her2 epitopes (competition analysis; binding analysis SPR). These Her2 binding proteins do not compete for Her2 binding and thus, use different or non-overlapping epitopes of Her2.
**FIG. 13** shows that the Her2 binding proteins Affilin-142628 and Affilin-143692 bind to different Her2 epitopes than Trastuzumab (Herceptin).

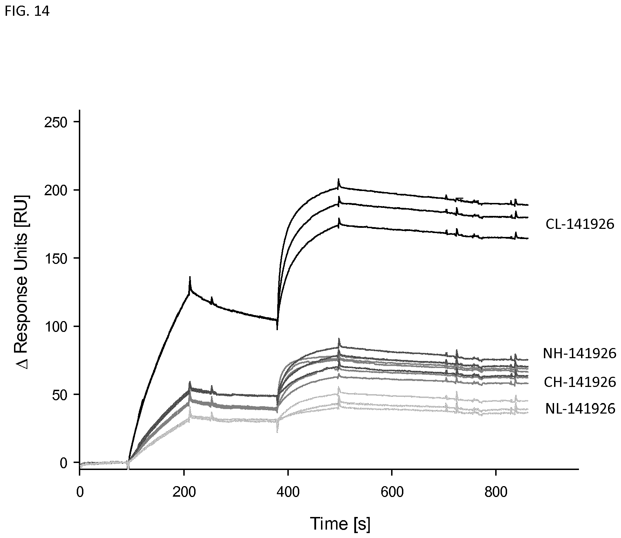

**FIG. 14** shows the simultaneous binding of a bispecific fusion protein to Her2 and EGFR. The fusion of a Her2 binding protein to an EGFR specific monoclonal antibody (Cetuximab) enables bispecific targeting, as shown for example for fusion proteins SEQ ID NOs: 44-47.

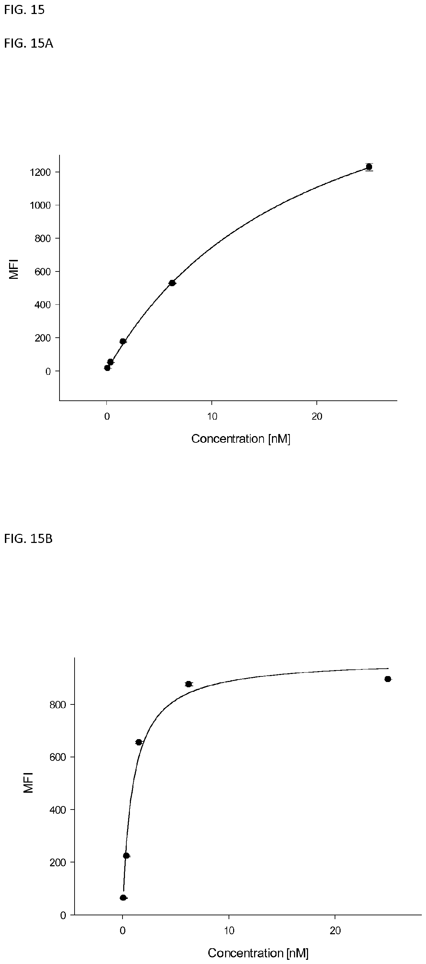

**FIG. 15** shows the flow cytometric binding analysis of a bispecific fusion protein comprising an Her2 specific Affilin fused to the C-terminus of the light chain of Cetuximab (CL-141926; SEQ ID NO: 44) on Her2 overexpressing CHO K1 cells (FIG. 15 B) and on EGFR overexpressing CHO K1 cells (FIG. 15 B). The fusion protein shows binding to both extracellular targets. The figure shows the median fluorescence intensity (MFI), representing the binding of the Affilin-antibody fusion protein to EGFR and to Her2 expressing cells at the indicated concentrations.

### Detailed Description of the Invention

Before the present invention is described in more detail below, it is to be understood that the present specification is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art to which this invention belongs.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variants such as "comprises" and "comprising", will be understood to imply the inclusion of a stated integer or step or group of integers or steps but not the exclusion of any other integer or step or group of integers or steps.

Several documents (for example: patents, patent applications, scientific publications, manufacturer's specifications, instructions, GenBank Accession Number sequence submissions etc.) are cited throughout the text of this application. Nothing herein is to be construed as an admission that the invention is not entitled to antedate such disclosure by virtue of prior invention. In the event of a conflict between the definitions or teachings of documents cited herein and definitions or teachings recited in the present specification, the text of the present specification takes precedence.

All sequences referred to herein are disclosed in the attached sequence listing that, with its whole content and disclosure, is a part of this specification.

### General definitions of important terms used in the specification

The terms "protein" and "polypeptide" refer to any chain of two or more amino acids linked by peptide bonds, and does not refer to a specific length of the product. Thus, "peptides", "protein", "amino acid chain," or any other term used to refer to a chain of two or more amino acids, are included within the definition of "polypeptide," and the term "polypeptide" may be used instead of, or interchangeably with any of these terms.

The term "polypeptide" is also intended to refer to the products of post-translational modifications of the polypeptide, including without limitation glycosylation, acetylation, phosphorylation, amidation, proteolytic cleavage, modification by non-naturally occurring amino acids and similar modifications which are well known in the art. Thus, binding proteins comprising two or more protein moieties also fall under the definition of the term "protein" or "polypeptides".

The term "ubiquitin" or "unmodified ubiquitin" refers to ubiquitin in accordance with SEQ ID NO: 1 and to proteins with at least 95 % identity, such as SEQ ID NO: 2 (point mutations in positions 45, 75, 76 which do not influence binding to a target), to a di-ubiquitin according to SED ID NO: 4 and to proteins with at least 95 % identity, such as , di-ubiquitin according to SEQ ID NO: 48, and according to the following definition. Particularly preferred are ubiquitin molecules from mammals, e.g. humans, primates, pigs, and rodents. On the other hand, the ubiquitin origin is not relevant since according to the art all eukaryotic ubiquitins are highly conserved and the mammalian ubiquitins examined up to now are even identical with respect to their amino acid sequence. In addition, ubiquitin from any other eukaryotic source can be used. For instance ubiquitin of yeast differs only in three amino acids from the wild-type human ubiquitin (SEQ ID NO: 1).

The term "di-ubiquitin" refers to a protein comprising two unmodified ubiquitin moieties linked to each other in head-to-tail orientation. An example is given in SED ID NO: 4 (point mutations in positions 45, 75, 76, 151, 152 of wildtype ubiquitin; these point mutations do not influence binding to a target; clone 139090), and in SEQ ID NO: 48. The amino acid sequence identity between SEQ ID NO: 4 and SEQ ID NO: 48 is 96.7 %. A di-ubiquitin as described herein is an artificial protein of 152 amino acids consisting of two ubiquitin moieties directly linked to each other without a peptide linker between the two ubiquitin moieties. A di-ubiquitin as understood herein is a protein with at least 95 % identity to SEQ ID NO: 4.

The terms "modified ubiquitin" and "ubiquitin mutein" and "Affilin" are all used synonymously and can be exchanged. The term "modified ubiquitin" or "ubiquitin mutein" or "Affilin" as used herein refers to derivatives of ubiquitin which differ from said unmodified ubiquitin by amino acid exchanges, insertions, deletions or any combination thereof, provided that the ubiquitin mutein has a specific binding affinity to a target epitope or antigen which is at least 10fold lower or absent in unmodified ubiquitin. This functional property of an ubiquitin mutein (Affilin; modified ubiquitin) is a *de novo* created function.

The term "Affilin®" (registered trademark of Scil Proteins GmbH) refers to non-immunoglobulin derived binding proteins based on ubiquitin muteins. An Affilin protein is not a natural ubiquitin existing in or isolated from nature, for example, as shown in SEQ ID NO: 1. The scope of the invention excludes unmodified ubiquitin. An Affilin molecule as described herein comprises, essentially consists, or consists of either two differently modified ubiquitin moieties linked together in a head-to-tail fusion or an Affilin molecule that comprises, essentially consists, or consists of one modified ubiquitin moiety. A "head-to-tail fusion" is to be understood as fusing two proteins together by connecting them in the direction (head) N-C-N-C- (tail) (tandem molecule), as described for example in EP2379581B1. The head part is designated as the first moiety and the tail part as the second moiety. In this head-to-tail fusion, two moieties may be connected directly without any linker (e.g. SEQ ID NOs: 5-38). Alternatively, the fusion of two proteins can be performed via linkers, for example, a polypeptide linker, as described herein.

The term "substitution" includes "conservative" and "non-conservative" substitutions. "Conservative substitutions" may be made, for instance, on the basis of similarity in polarity, charge, size, solubility, hydrophobicity, hydrophilicity, and/or the amphipathic nature of the amino acid residues involved. Amino acids can be grouped into the following standard amino acid groups: (1) hydrophobic side chains: Ala (A), Met (M), Leu (L), Val (V), lie (I); (symbol "H" in Figure 1) (2) acidic polar side chain: Asp (D), Glu (E) (symbol "-" in Figure 1); (3) basic side chain polarity: Lys (K), Arg (R), His (H) (symbol "+" in Figure 1); (4) aromatic amino acids: Trp (W), Tyr (Y), Phe (F) (symbol "o" in Figure 1); (5) polar amino acids: Thr (T), Ser (S), Asn (N), Gln (Q) (symbol "wave" in Figure 1); (6) residues that influence chain orientation: Gly (G), Pro (P); and (7) Cys (C). As used herein, "conservative substitutions" are defined as exchanges of an amino acid by another amino acid listed within the same group of the standard amino acid groups shown above. For example, the exchange of Asp by Glu retains one negative charge in the so modified polypeptide. In addition, Gly and Pro may be substituted for one another based on their ability to disrupt α-helices. Some preferred conservative substitutions within the above groups are exchanges within the following sub-groups: (i) Ala, Val, Leu and Ile; (ii) Ser and Thr; (ii) Asn and Gln; (iv) Lys and Arg; and (v) Tyr and Phe. Given the known genetic code, and recombinant and synthetic DNA techniques, the skilled scientist can readily construct DNAs encoding the conservative amino acid variants.

As used herein, "non-conservative substitutions" or "non-conservative amino acid exchanges" are defined as exchanges of an amino acid by another amino acid listed in a different group of the amino acid groups (1) to (7) shown above.

The term "insertions" comprises the addition of amino acids to the original amino acid sequence of ubiquitin wherein the ubiquitin remains stable without significant structural change. Naturally, loop regions connect regular secondary structure elements. The structure of human unmodified ubiquitin (SEQ ID NO: 1) reveals six loops at amino acid regions 8-11, 17-22, 35-40, 45-47, and 50-63 which connect secondary structure elements such as beta sheets and alpha helix. In one embodiment of the invention, Her2 binding proteins are disclosed comprising a ubiquitin mutein having a combination of an insertion and substitutions. In one embodiment, ubiquitin muteins have insertions of 2-10 amino acid residues, preferably within the most N-terminal loop within amino acids 8-11. Specifically, the number of amino acid residues to be inserted is 2, 3, 4, 5, 6, 7, 8, 9, 10, preferably 2 - 10 amino acid residues, most preferred 6-9 amino acid residues.

The term "antibody" as used in the context of the present invention comprises monoclonal antibodies having two heavy chains and two light chains (immunoglobulin or IgG antibodies). Furthermore, also fragments or derivatives thereof, which still retain the binding specificity, are comprised in the term "antibody". The term "antibody" also includes embodiments such as chimeric (human constant domain, non-human variable domain), single chain and humanized (human antibody with the exception of non-human CDRs) antibodies. Full-length IgG antibodies consisting of two heavy chains and two light chains are most preferred in the context of the present invention. Heavy and light chains are connected via non-covalent interactions and disulfide bonds.

In the present specification, the terms "target antigen", "target", "antigen" and "binding partner" are all used synonymously and can be exchanged. Preferably the target is one of the targets defined herein below. The term "antigen", as used herein, is to be interpreted in a broad sense and includes any target moiety that is bound by the binding moieties of the binding proteins of the present invention.

The terms "protein capable of binding" or "binding protein" or "binding Her2" or "binding affinity for" as used in the context of the present invention refer to a protein comprising a binding capability to a defined target antigen.

The term "Her2 binding protein" refers to a protein with high affinity binding capability to Her2.

An "antigen binding site" refers to the site, i.e. one or more amino acid residues, of an antigen binding molecule which provide interaction with the antigen. A native immunoglobulin molecule typically has two antigen binding sites, a Fab molecule typically has a single antigen binding site.

The term "epitope" includes any molecular determinant capable of being bound by an antigen binding protein as defined herein and is a region of a target antigen that is bound by an antigen binding protein that targets that antigen, and when the antigen is a protein, it may include specific amino acids that directly contact the antigen binding protein. In a conformational epitope, amino acid residues are separated in the primary sequence, but are located near each other on the surface of the molecule when the polypeptide folds into the native three-dimensional structure. A linear epitope is characterized by two or more amino acid residues which are located adjacent in a single linear segment of a protein chain. In other cases, the epitope may include determinants from posttranslational modifications of the target protein such as glycosylation, phosphorylation, sulfatation, acetylation, fatty acids or others.

The term "fused" means that the components (e.g. an Affilin molecule and a monoclonal antibody or a Fab fragment) are linked by peptide bonds, either directly or via peptide linkers.

The term "fusion protein" relates to a protein comprising at least a first protein joined genetically to at least a second protein. A fusion protein is created through joining of two or more genes that originally coded for separate proteins. Thus, a fusion protein may comprise a multimer of different or identical binding proteins which are expressed as a single, linear polypeptide. It may comprise one, two, three or even more first and/or second binding proteins. A fusion protein as used herein comprises at least a first binding protein (e.g. Affilin) which is fused with at least a second binding protein, e.g. a monoclonal antibody or a fragment thereof. Such fusion proteins may further comprise additional domains that are not involved in binding of the target, such as but not limited to, for example, multimerization moieties, polypeptide tags, polypeptide linkers.

The term "conjugate" as used herein relates to a protein comprising or essentially consisting of at least a first protein attached chemically to other substances such as to a second protein or a non-proteinaceous moiety.

The conjugation can be performed by means of organic synthesis or by use of enzymes including natural processes of enzymatic post-translational modifications. Examples for protein conjugates are glycoproteins (conjugated protein with carbohydrate component) or lipoproteins (conjugated protein with lipid component).

The molecule can be attached e.g. at one or several sites through any form of a linker. Chemical coupling can be performed by chemistry well known to someone skilled in the art, including substitution (e.g. N-succinimidyl chemistry), addition or cycloaddition (e.g. maleimide chemistry or click chemistry) or oxidation chemistry (e.g. disulfide formation). Some examples of non-proteinaceous polymer molecules which are chemically attached to Her2 binding proteins of the invention are hydroxyethyl starch, polyethylene glycol, polypropylene glycol, dendritic polymers, or polyoxyalkylene and others.

A fusion protein or protein conjugate may further comprise one or more reactive groups or peptidic or non-peptidic moieties such as ligands or therapeutically or diagnostically relevant molecules such as radionuclides or toxins. It may also comprise small organic or non-amino acid based compounds, e.g. a sugar, oligo- or polysaccharide, fatty acid, etc. Methods for attaching a protein of interest to such non-proteinaceous components are well known in the art, and are thus not described in further detail here.

The terms "bispecific binding molecule" or "multispecific binding molecule" mean that the antigen binding molecule is able to specifically bind two or multiple different epitopes. Typically, a bispecific antigen binding molecule comprises two antigen binding sites, each of which is specific for a different epitope. In certain embodiments the bispecific antigen binding molecule is capable of simultaneously binding two epitopes, particularly two epitopes expressed on two distinct cells. The term "bispecific binding molecule" or "bispecific binding protein" means that Her2 binding proteins of the present invention are capable of specifically binding to two different epitopes. Moreover, the bispecific binding molecule of the present invention is capable of binding to two different epitopes at the same time. This means that a bispecific construct is capable of simultaneously binding to at least one epitope "A" and at least one epitope "B", wherein A and B are not the same. The two epitopes may be located on the same or different target antigens which means that the fusion molecules of the present invention can bind one target at two different epitopes or two target antigens each with its own epitope. Similarly, "multispecific binding molecules" are capable of binding multiple epitopes at the same time wherein the epitopes may be located on the same or different antigens.

Alternatively, said binding proteins may bind to different, non-overlapping epitopes on the same or different target molecules and are thus classified as bispecific, trispecific, multispecific, etc., for example αβ, βγ, αδ, αβγ, αβγδ binding to epitopes AB, BC, AD, ABC or ABCD, respectively. For example, fusion proteins with Her2-specific Affilin and anti-EGFR-monoclonal antibody are bispecific.

The term "multimeric binding molecules" refers to fusion proteins that are multivalent and / or multispecific, comprising two or more moieties (i.e. bivalent or multivalent) of binding protein α, β and/or γ etc., e.g. αα, βββ, ααβ, ααββ, αγγ, ββγ, αβγδδ, etc.. For example, ααβγ is trispecific and bivalent with respect to epitope A.

For example, the fusion proteins of Her2-specific Affilin and monoclonal antibodies as described herein are at least "bivalent" because they comprise at least two binding proteins (for example, an Affilin and an monoclonal antibody).

Said binding proteins may bind specifically to the same or overlapping epitopes on a target antigen (monospecific), e.g. the composition of the binding protein may be described by (α)₂, (α)₃, (α)₄, (β)₂, (β)₃, (β)₄ etc.. In this case, the fusion molecules are monospecific but bivalent, trivalent, tetravalent, or multivalent for the epitope A or epitope B, respectively.

The term "amino acid sequence identity" refers to a quantitative comparison of the identity (or differences) of the amino acid sequences of two or more proteins. "Percent (%) amino acid sequence identity" with respect to a reference polypeptide sequence is defined as the percentage of amino acid residues in a sequence that are identical with the amino acid residues in the reference polypeptide sequence, after aligning the sequences and introducing gaps, if necessary, to achieve the maximum percent sequence identity.

To determine the sequence identity, the sequence of a query protein is aligned to the sequence of a reference protein, for example, to SEQ ID NO: 4 (di-ubiquitin) or to SEQ ID NO: 1 (ubiquitin). Methods for alignment are well known in the art. For example, for determining the extent of an amino acid sequence identity of an arbitrary polypeptide relative to the amino acid sequence of SEQ ID NO: 4 or SEQ ID NO: 1, the SIM Local similarity program is preferably employed (Xiaoquin Huang and Webb Miller (1991), Advances in Applied Mathematics, vol. 12: 337-357), that is freely available (see also: http://www.expasy.org/tools/sim-prot.html). For multiple alignment analysis ClustalW is preferably used (Thompson et al. (1994) Nucleic Acids Res., 22(22): 4673-4680).

In the context of the present invention, the extent of sequence identity between a modified sequence and the sequence from which it is derived (also termed "parental sequence") is generally calculated with respect to the total length of the unmodified sequence, if not explicitly stated otherwise. Each amino acid of the query sequence that differs from the reference amino acid sequence at a given position is counted as one difference.

An insertion or deletion in the query sequence is also counted as one difference. For example, an insertion of a linker between two ubiquitin moieties is counted as one difference compared to the reference sequence. The sum of differences is then related to the length of the reference sequence to yield a percentage of non-identity.

The quantitative percentage of identity is calculated as 100 minus the percentage of non-identity. In specific cases of determining the identity of ubiquitin muteins aligned against unmodified ubiquitin, differences in positions 45, 75 and/or 76 are not counted, in particular, because they are not relevant for the novel binding capability of the ubiquitin mutein. The ubiquitin moiety can be modified in amino acid residues 45, 75 and/or 76 without affecting its binding capability; said modifications might, however, be relevant for achieving modifications in the biochemical properties of the mutein. Generally, a ubiquitin used as starting material for the modifications has an amino acid identity of at least 95 %, of at least 96 % or of at least 97 %, or of at least an amino acid sequence identity of 98 % to SEQ ID NO: 1. Thus, a polypeptide which is, for example, 95 % "identical" to a reference sequence may comprise, for example, five point mutations or four point mutations and one insertion etc., per 100 amino acids, compared to the reference sequence.

The term "dissociation constant" or "K_{D}" defines the specific binding affinity. As used herein, the term "K_{D}" (usually measured in "mol/L", sometimes abbreviated as "M") is intended to refer to the dissociation equilibrium constant of the particular interaction between a first compound and a second compound. In the context of the present invention, the term K_{D} is particularly used to describe the binding affinity between a Her2-binding protein and Her2. A high affinity corresponds to a low value of K_{D}. Thus, the expression "a K_{D} of at least e.g. 10⁻⁷ M" means a value of 10⁻⁷ M or lower (binding more tightly). 1 x 10⁻⁷ M corresponds to 100 nM. A value of 10⁻⁵ M and below down to 10⁻¹² M can be considered as a quantifiable binding affinity. Depending on the application a value of 10⁻⁷ to 10⁻¹² M is preferred for chromatographic applications or for diagnostic or therapeutic applications. In accordance with the invention the affinity for the target binding is in the range of 7 x 10⁻⁷ M (700 nM) or less.

Final target binding affinity can be ideally 10⁻⁹ M (1 nM) or less.

Binding proteins of the invention comprise two ubiquitin muteins linked directly without any linker to result in unique and high affinity Her2 binding proteins with substitutions at least in 12, 13, or 14 positions selected from 42, 44, 68, 70, 72, 73, 74, 82, 84, 138, 139, 140, 141, and 142 of di-ubiquitin (SEQ ID NO: 4 or SEQ ID NO: 48), and optionally in 0, 1, 2, 3, 4, 5, or 6 further substitutions.

Binding proteins of the invention can be fused, e.g. genetically, to other functional protein moieties. In the context of such fusion proteins of the invention the term "linker" refers to a single amino acid or a polypeptide that joins at least two other protein molecules covalently. The linker is e.g. genetically fused to the first and second protein or protein moieties to generate a single, linear polypeptide chain. The length and composition of a linker may vary between at least one and up to about 50 amino acids. Preferably, the linker length is between one and 30 amino acids. More preferably, the peptide linker has a length of between 1 and 20 amino acids; e.g. 1, 2, 3, 4, 5, 6, 7, 8, 9, 10, 11, 12, 13, 14, 15, 16, 17, 18, 19, or 20 amino acids. It is preferred that the amino acid sequence of the peptide linker is not immunogenic to human beings, stable against proteases and optionally does not form a secondary structure. An example is a linker comprised of small amino acids such as glycine or serine. The linkers can be glycine-rich (e.g., more than 50 % of the residues in the linker can be glycine residues). Preferred are glycine-serine-linkers of variable length consisting of glycine and serine residues only. In general, linkers of the structure (SGGG)ₙ or permutations of SGGG, e.g. (GGGS)ₙ, can be used wherein n can be any number between 1 and 6, preferably 1 or 2 or 3. Also preferred are linkers comprising further amino acids. Other linkers for the genetic fusion of proteins are known in the art and can be used. In one embodiment of the invention, the first binding protein (e.g. Affilin) and the second binding protein (e.g. monoclonal antibody or fragment thereof) are linked via a (G₃S)₄ linker.

In case of chemical conjugates of the binding proteins of the invention, the term "linker" refers to any chemical moiety which connects the Her2 binding protein with other proteinaceous or non-proteinaceous moieties either covalently or non-covalently, e.g., through hydrogen bonds, ionic or van der Waals interactions, such as two complementary nucleic acid molecules attached to two different moieties that hybridize to each other, or chemical polymers such as polyethylene glycol or others. Such linkers may comprise reactive groups which enable chemical attachment to the protein through amino acid side chains, the N-terminal α-amino- or C-terminal carboxy-group of the protein. Such linkers and reactive groups are well-known to those skilled in the art and not described further.

Her2 (Human Epidermal Growth Factor Receptor 2; synonym names are ErbB-2, Neu, CD340 or p185) is a 185-kDa receptor first described in 1984 (Schlechter et al (1984) Nature 312:513-516). Amplification or overexpression of this gene has been shown to play an important role in the pathogenesis and progression of certain aggressive types of breast cancer, and Her2 is known as an important biomarker and target of therapy for the disease. Other tumors where Her2 plays a role include ovarian cancer and gastric cancer. Human Her2 is represented by the NCBI accession number NP_004439; the extracellular domain (residues 1-652) of Her2 is represented by the uniprot Accession Number p04626. The term "Her2" comprises all polypeptides which show a sequence identity of at least 70 %, 80 %, 85 %, 90 %, 95 %, 96 % or 97 % or more, or 100 % to NP_004439 and have the functionality of Her2.

### Detailed description of the embodiments of the invention

The Her2 binding protein of the invention comprises, essentially consists of or consists of two differently modified ubiquitin moieties directly connected without a linker in head-to-tail orientation. Furthermore, the Her2 binding protein of the invention has an amino acid identity of at least 85 % to di-ubiquitin (SEQ ID NO: 4); i.e. a maximum of 23 amino acids are modified in di-ubiquitin (SEQ ID NO: 4) (152 amino acids total) to generate a novel binding property of di-ubiquitin (SEQ ID NO: 4) to Her2. Preferred amino acid identities of the novel Her2 binding proteins are at least 86 %, at least 87 % (corresponding to 20 amino acids modified), at least 88 %, or at least 89 %, at least 90 % (corresponding to 15 amino acids modified), at least 91 % (corresponding to 14 amino acids modified), at least 92 % (corresponding to 12 amino acids modified) to di-ubiquitin (SEQ ID NO: 4). Thus, Her2 binding proteins of the invention show 85 % to 92 % identity to di-ubiquitin, more preferably between 87 % to 91 % identity to di-ubiquitin.

The Her2 binding protein of the invention with binding affinity (K_{D}) of less than 700 nM for Her2 comprises, essentially consists, or consists of an amino acid sequence according to di-ubiquitin having the amino acid sequence of SEQ ID NO: 4, wherein amino acids selected from at least 12, 13, or 14 amino acids selected from positions R42, 144, H68, V70, R72, L73, R74, K82, L84, Q138, K139, E140, S141, and T142 of di-ubiquitin (SEQ ID NO: 4) are substituted, wherein the Her2 binding protein has at least 85 % sequence identity to di-ubiquitin (SEQ ID NO: 4), and wherein the amino acid at
position 42 is substituted by a polar amino acid,
position 44 is substituted by a hydrophobic or polar amino acid,
position 68 is substituted by an aromatic amino acid,
position 70 is substituted by an aromatic amino acid,
position 72 is substituted by a polar or aromatic amino acid,
position 73 is substituted by any amino acid but not basic or acidic amino acid,
position 74 is substituted by an aromatic, basic or polar amino acid,
position 82 is substituted by any amino acid but not basic or acidic amino acid,
position 84 is substituted by a basic or acidic amino acid,
position 138 is substituted by a basic or acidic or polar amino acid,
position 139 is substituted by an acidic or hydrophobic amino acid or glycine,
position 140 is substituted by an aromatic amino acid,
position 141 is substituted by a hydrophobic or polar or basic amino acid, and/or
position 142 is substituted by a hydrophobic or polar amino acid. The Her2 binding proteins as described in this invention show not more than 92 % sequence identity to SEQ ID NO: 4. The Her2 binding proteins comprise 152 amino acids with at least 85 % to di-ubiquitin (SEQ ID NO: 4), provided that at least 12, 13, or 14 amino acids selected from positions R42,144, H68, V70, R72, L73, R74, K82, L84, Q138, K139, E140, S141, and T142 are substituted. All Her2 binding proteins have substitutions in positions R42, V70, R72, L73, K82, L84, Q138, K139, E140, and T142, and in positions I44, H68, R74, and S141. Surprisingly, the specific combination of substitutions in said 12, 13, or 14 positions of SEQ ID NO: 4 results in high affinity Her2 binding proteins.

These proteins are artificial proteins that are created *de novo.* The Her2 binding proteins of the invention do not exist in nature. Examples for *de novo* created Her2 binding proteins are provided in SEQ ID NOs: 5-38.

The Her2 binding protein is substituted in at least 12 positions selected from positions 42, 44, 68, 70, 72, 73, 74, 82, 84, 138, 139, 140, 141, and 142 of di-ubiquitin (SEQ ID NO: 4) and has no further substitution, for example, SEQ ID NOs: 29, 33, one additional substitution, for example, SEQ ID NOs: 27, 28, 31, 32, two additional substitutions, for example, SEQ ID NOs: 14, 16, 21, 25, 26, 30, 35, three additional substitutions, for example, SEQ ID NOs: 6, 12, 13, 15, 17, 18, 20, 34, 36, four additional substitutions, for example, SEQ ID NOs: 10, 11, 19, 22, 23, 24, five additional substitutions, for example, SEQ ID NOs: 5, 7, 8, 9, or six additional substitutions, for example, SEQ ID NO: 37. For example, further 1, 2, 3, 4, 5, or 6 substitutions in addition to the at least 12 substitutions in positions 42, 44, 68, 70, 72, 73, 74, 82, 84, 138, 139, 140, 141, and 142 of SEQ ID NO: 4 may be preferably selected from positions 6, 10, 11, 15, 20, 21, 23, 27, 28, 31, 34, 36, 40, 46, 48, 49, 52, 58, 62, 63, 75, 78, 88, 92, 95, 96, 98, 114, 120, 124, 131, 133, 144, and/or 147 of SEQ ID NO: 4 (see Figure 1 and Table 1).

**Table 1. Her2 binding proteins of the invention - number of substitutions and degree of identity to SEQ ID NO: 4**

| SEQ ID NO: | Number of substitutions in positions 42, 44, 68, 70, 72, 73, 74, 82, 84, 138, 139, 140, 141, and 142 | Number of additional substitutions | total number of substitutions | % identity to SEQ ID NO: 4 |
|---|---|---|---|---|
| 5 | 14 | 6 | 20 | 86.8 |
| 7 | 14 | 6 | 20 | 86.8 |
| 8 | 14 | 6 | 20 | 86.8 |
| 9 | 14 | 6 | 20 | 86.8 |
| 10 | 14 | 5 | 19 | 87.5 |
| 11 | 14 | 5 | 19 | 87.5 |
| 19 | 14 | 5 | 19 | 87.5 |
| 22 | 14 | 5 | 19 | 87.5 |
| 23 | 14 | 5 | 19 | 87.5 |
| 24 | 14 | 5 | 19 | 87.5 |
| 12 | 14 | 4 | 18 | 88.2 |
| 13 | 14 | 4 | 18 | 88.2 |
| 15 | 14 | 4 | 18 | 88.2 |
| 17 | 14 | 4 | 18 | 88.2 |
| 18 | 14 | 4 | 18 | 88.2 |
| 20 | 14 | 4 | 18 | 88.2 |
| 14 | 14 | 3 | 17 | 88.9 |
| 16 | 14 | 3 | 17 | 88.9 |
| 21 | 14 | 3 | 17 | 88.9 |
| 25 | 14 | 3 | 17 | 88.9 |
| 26 | 14 | 3 | 17 | 88.9 |
| 30 | 14 | 3 | 17 | 88.9 |
| 25 | 14 | 3 | 17 | 88.9 |
| 6 | 13 | 4 | 17 | 88.9 |
| 36 | 13 | 4 | 17 | 88.9 |
| 27 | 14 | 2 | 16 | 89.5 |
| 28 | 14 | 2 | 16 | 89.5 |
| 32 | 14 | 2 | 16 | 89.5 |
| 34 | 12 | 4 | 16 | 89.5 |
| 29 | 14 | 1 | 15 | 90.3 |
| 31 | 13 | 2 | 15 | 90.3 |
| 33 | 12 | 1 | 13 | 91.4 |

Many examples of Her2 binding proteins according to the invention are disclosed herein (see, for example, Figure 1a, SEQ ID NOs: 5-38). The Her2 binding Affilin molecules of the invention bind to the isolated extracellular domain of Her2 with measurable binding affinity of less than 700 nM, less than 500 nM, less than 100 nM, less than 20 nM, less than 10 nM (for example, SEQ ID NOs: 6, 14, 15, 18, 22, 24, 25, 26, 28, 35, 36, 38), and more preferred less than 1 nM (for example, SEQ ID NOs: 7, 8, 9, 10, 11, 12, 13, 16, 17, 19, 20, 23) (binding affinity as determined by Biacore; see, for example, Figure 2). The di-ubiquitin (SEQ ID NO: 4) does not naturally bind to Her2 with any measurable binding affinity. All Her2 binding proteins of the invention show *de novo* created binding to Her2 with high affinity.

Substitutions of the Her2 binding protein based on di-ubiquitin (SEQ ID NO: 4) are substitutions of amino acids selected from position 70 and 140 by aromatic amino acids, position 42 by a polar amino acid, position 44 is substituted by a hydrophobic or polar amino acid, position 68 is substituted by an aromatic amino acid, position 72 is substituted by a polar or aromatic amino acid, position 73 is substituted by any amino acid but not basic or acidic amino acid, position 74 is substituted by an aromatic, basic or polar amino acid, position 82 is substituted by any amino acid but not basic or acidic amino acid, position 84 is substituted by a basic or acidic amino acid, position 138 is substituted by a basic or acidic or polar amino acid, position 139 is substituted by acidic or hydrophobic amino acid or Glycine, position 141 is substituted by hydrophobic or polar or basic amino acid, and/or position 142 is substituted by a hydrophobic or polar amino acid. Preferred substitutions of the Her2 binding protein based on di-ubiquitin (SEQ ID NO: 4) are selected from R42T, R42S, R42L, I44A, I44V, I44S, I44T, H68W, H68Y, H68F, V70Y, V70W, R72T, R72F, R72G, R72Y, L73W, L73S, L73V, L73I, R74Y, R74S, R74N, R74K, K82T, K82L, K82N, K82I, K82Y, L84H, L84D, L84E, L84S, Q138S, Q138R, Q138E, K139E, K139G, K139L, E140W, S141A, S141R, T142I, T142L, and/or T142N. Further preferred are Her2 binding proteins with a specific combination of amino acid substitutions in SEQ ID NO: 4, for example, at least R42T, I44A, H68W, V70Y, R72T, L73W, R74Y, K82T, L84H, as for example, in SEQ ID NOs: 7-29 and 38.

Other preferred Her2 binding proteins with a specific combination of amino acid substitutions in di-ubiquitin (SEQ ID NO: 4), are for example at least R42S, I44V, H68Y, V70Y, R72F, L73S, K82L, L84D, as for example, in SEQ ID NOs: 34, 35, 36, and 37. Further preferred are Her2 binding proteins with a specific combination of amino acid substitutions in di-ubiquitin (SEQ ID NO: 4), for example, Q138S, K139E, E140W, S141A, T142I (for example, in SEQ ID NOs: 5, 7-29, 33, 36, 37), or Q138R, K139G, E140W, T142L (for example, in SEQ ID NOs: 6, 34, 35), or Q138E, K139L, E140W, S141R, T142N (for example, in SEQ ID NOs: 30, 31, 32).

In preferred embodiments, Her2 binding proteins of the invention comprise amino acid sequences selected from the group consisting of SEQ ID NO: 5-38. Her2 binding proteins described herein may comprise amino acid sequences that exhibit at least 85 % or at least 87 % or at least 91 % or at least 94 % or at least 96 % sequence identity to one or more of the amino acid sequences of SEQ ID NO: 5-38. Figure 1 shows examples for Her2 binding proteins of the invention.

In further embodiments, the Her2 binding protein based on SEQ ID NO: 1 comprises an insertion of amino acids within a natural loop region, preferably within the first loop of the N-terminal part, in addition to the substitutions in positions 62, 63, 64, 65, 66 of SEQ ID NO: 1 and possibly further 1, 2, 3, 4, 5, or 6 modifications, for example in positions 2, 4, 6, or 8. A preferred Her2 binding protein based on SEQ ID NO: 1 has substitutions in amino acid region 62 - 66 of SEQ ID NO: 1 combined with an insertion of 2 - 10 amino acids, preferably 4 - 9 amino acids, even more preferred 6, 7, 8, or 9 amino acids, in a natural loop region of said SEQ ID NO: 1, preferably in region 8 -11, more preferably between position 9 and 10 corresponding to SEQ ID NO: 1. For example, Her2 binding Affilin-144567 (SEQ ID NO: 39) has an insertion of 6 amino acids (PYETQV, SEQ ID NO: 42) at position 9 of SEQ ID NO: 1 in addition to substitutions in positions 2, 4, 6, 62, 63, 64, 65, 66 SEQ ID NO: 1 (2R, 4G, 6G, 62R, 63F, 64W, 65K, 66K). Her2 binding Affilin-143692 (SEQ ID NO: 40) has an insertion of 9 amino acids (AGNPSHMHH, SEQ ID NO: 43) at position 9 of SEQ ID NO: 1 in addition to substitutions in positions 2, 4, 6, 62, 63, 64, 65, 66 of SEQ ID NO: 1 (2D, 4D, 6M, 62H, 63W, 641, 65L, 66N). In preferred embodiments, Her2 binding proteins of the invention comprise amino acid sequences selected from the group consisting of SEQ ID NO: 39 and SEQ ID NO: 40. Her2 binding proteins described herein may comprise amino acid sequences that exhibit at least 85 % or at least 87 % or at least 91 % or at least 94 % or at least 96 % sequence identity to one or more of the amino acid sequences of SEQ ID NOs: 39-40.

The further characterization of Her2 binding proteins can be performed in the form of soluble proteins. The appropriate methods are known to those skilled in the art or described in the literature. The methods for determining the binding affinities are known *per se* and can be selected for instance from the following methods known in the art: Surface Plasmon Resonance (SPR) based technology, Bio-layer interferometry (BLI), enzyme-linked immunosorbent assay (ELISA), flow cytometry, fluorescence spectroscopy techniques, isothermal titration calorimetry (ITC), analytical ultracentrifugation, radioimmunoassay (RIA or IRMA) and enhanced chemiluminescence (ECL). Some of the methods are described in the Examples below.

For stability analysis, for example spectroscopic or fluorescence-based methods in connection with chemical or physical unfolding are known to those skilled in the art. Exemplary methods for characterization of Her2 binding proteins are outlined in the Examples section of the present specification.

For example, the biochemical target binding analysis is summarized in Figure 2 and further described in the Examples. All binding proteins of the invention have an affinity of less than 700 nM for Her2, as determined by SPR based technology. In an embodiment of the first aspect, the Her2-binding protein has a dissociation constant K_{D} to human Her2 in the range between 0.01 nM and 700 nM, more preferably between 0.05 nM and 500 nM, more preferably between 0.1 nM and 100 nM, more preferably between 0.1 nM and 20 nM, more preferably between 0.1 nM and 10 nM. The dissociation constant K_{D} can be determined by ELISA or by surface plasmon resonance assays. Typically, the dissociation constant K_{D} is determined at 20°C, 25°C, or 30°C. If not specifically indicated otherwise, the K_{D} values recited herein are determined at 25°C by surface plasmon resonance.

In addition, temperature stability was determined by differential scanning fluorimetry (DSF), as described in further detail in the Examples and as shown in Figure 2. In addition to results shown in Figure 2, solubility of at least 80 % was confirmed for all Her2 binding molecules by size exclusion chromatography; no Her2 binding molecule of the invention shows aggregation. Figure 3 shows binding kinetics for two different Her2 binding proteins.

Competitive binding experiments comparing Affilin molecules show that the epitope that is bound by different Her2 binding proteins, for example Affilin-142628 and Affilin-143692, is not identical or non-overlapping (see Figure 12). These Her2 binding proteins do not compete for Her2 binding. Further, Her2 binding proteins bind to different Her2 epitopes than the monoclonal antibody Trastuzumab. Figure 13 shows that Affilin-142628 and Affilin-143692 bind to different or non-overlapping Her2 epitopes than Trastuzumab. In addition, Affilin-141926 (SEQ ID NO: 28), Affilin-141884 (SEQ ID NO: 38), Affilin-141890 (SEQ ID NO: 30), and Affilin-141975 (SEQ ID NO: 37) bind to different or non-overlapping epitopes of Her2 than Trastuzumab (Table 2). The first K_{D} shown in Table 2 shows binding to Her2, the second K_{D} in the Table 2 shows binding to Her2 in the presence of Trastuzumab. Since both values are almost identical, it can be concluded that Affilin-proteins bind to different or non-overlapping epitops than Trastuzumab. In contrast, similar or overlapping epitopes with Trastuzumab show Affilin-141931 (SEQ ID NO: 27), Affilin-141912 (SEQ ID NO: 31), and Affilin-141935 (SEQ ID NO: 32).

**Table 2. Competition of Affilin-proteins with Trastuzumab**

| Affilin- | K_{D} (nM) | K_{D} (nM) |
|---|---|---|
| 141884 | 4.9 | 4.6 |
| 141890 | 24.3 | 25.5 |
| 141926 | 6.5 | 8.9 |
| 141975 | 41.2 | 39.4 |

Additional functional characterization was performed by cellular Her2 binding analysis with Her2 overexpressing cells, for example SkBr3 cells and genetically engineered CHO-K1 cells. Different concentrations of the Affilin molecules were tested. Her2 cell target binding was confirmed, as shown in Figures 4-9.

Furthermore, Affilin binding proteins show binding to Her2 on tumor tissue from cells of human origin (see Figure 10 and Figure 11). In particular and surprisingly, Affilin molecules show strong binding to Her2 expressed on SKOV-3 tumor tissue. No binding was observed on tissue from lung, liver, heart muscle, and ovary.

One embodiment of the invention covers a Her2 binding protein of the invention and further at least one additional protein or molecule. The additional protein can be a second binding protein with identical or different specificity for an antigen as the first binding protein. One embodiment of the invention covers a fusion protein or a conjugate comprising an Affilin-antibody fusion protein or conjugate, optionally further fused with or conjugated to a moiety, preferably selected from at least one member of the groups (i), (ii) and (iii) consisting of (i) a moiety modulating pharmacokinetics selected from a polyethylene glycol (PEG), a human serum albumin (HSA), a human serum albumin, an albumin-binding peptide, or an immunoglobulin (Ig) or Ig fragments, a polysaccharide, and, (ii) a therapeutically active component, optionally selected from a monoclonal antibody or a fragment thereof, a cytokine, a chemokine, a cytotoxic compound, an enzyme, or derivatives thereof, or a radionuclide, and (iii) a diagnostic component, optionally selected from a fluorescent compound, a photosensitizer, a tag, an enzyme or a radionuclide.

The conjugate molecule can be attached e.g. at one or several sites through a peptide linker sequence or a carrier molecule.

Further conjugation with proteinaceous or non-proteinaceous moieties to generate protein conjugates according to the invention can be performed applying chemical methods well-known in the art. In particular, coupling chemistry specific for derivatization of cysteine or lysine residues is applicable. In case of the introduction of non-natural amino acids further routes of chemical synthesis are possible, e.g. "click chemistry" or aldehyde specific chemistry and others.

Conjugates thus obtained can be selected from one or more of the following examples: (i) conjugation of the protein via lysine residues; (ii) conjugation of the protein via cysteine residues via maleimide chemistry; in particular, cysteine residues can be specifically introduced and can be located at any position suitable for conjugation of further moieties, (iii) peptidic or proteinogenic conjugations. These and other methods for covalently and non-covalently attaching a protein of interest to other functional components are well known in the art, and are thus not described in further detail here.

A further embodiment relates to Her2 binding proteins according to the invention, further comprising a moiety modulating pharmacokinetics or biodistribution, preferably selected from PEG, HSA, or an Ig or Ig fragments, for example an Fc fragment. Several techniques for producing proteins with extended half-life are known in the art.

The Her2 binding protein of the invention may also comprise a second binding protein which comprises or consists of a monoclonal antibody or fragment thereof. In one embodiment, the second binding protein is a monoclonal antibody with specificity for EGFR. It was surprisingly found that a bispecific binding molecule consisting of an EGFR monoclonal antibody and a Her2-specific Affilin is able to bind specifically to both EGFR and Her2. The EGFR binding level of the fusion protein is surprisingly higher than the EGFR-binding level of Cetuximab.

In some embodiments of the invention, bispecific binding molecules are provided comprising polypeptides specifically binding to Her2 and to EGFR simultaneously. Figure 14 shows the simultaneous binding of bispecific Affilin-antibody binding proteins to both target antigens (Her2 and EGFR). Figure 15 shows the flow cytometric binding analysis of Affilin-antibody binding proteins (e.g. C-terminal fusion to light chain; CL-141926, SEQ ID NO: 44) on Her2 overexpressing cells (Figure 15 a) and on EGFR overexpressing cells (Figure 15 b).

The figure shows the mean fluorescence intensity, representing the concentration dependent binding of the Affilin-antibody fusion protein to Her2 and to EGFR overexpressing cells.

In a further aspect of the invention, a Her2 binding protein or fusion protein or conjugate according to the invention is used in medicine, in particular in a method of medical treatment or diagnosis, preferably in cancer.

The membrane protein Her2 is known to be upregulated in tumor cells, resulting in uncontrolled growth of tumor cells and in the formation of metastases. New therapies for cancer patients include an inhibition of Her2 by targeted therapeutics such as for example the monoclonal antibodies Trastuzumab (Herceptin®) or Pertuzumab (Perjeta®). T-DM1, an antibody-drug conjugate, is highly effective against breast, uterine, and ovarian carcinosarcoma overexpressing Her2.

Overexpression of Her2 has been described in a wide variety of cancers. For example, overexpression of Her2 occurs in approximately 15 % to 30 % of breast cancers and 10 % to 30 % of gastric/gastroesophageal cancers, and has also been observed in other cancers like ovary, endometrium, bladder, lung colon, head and neck. Thus, the pharmaceutical composition comprising the Her2 binding protein of the invention, can be used for treatment of cancer in which Her2 is relevant for the development of the disease including but not limited to particularly breast, ovarian, gastric, but also in lung, head and neck, cervical, prostate, pancreas, and others.

The compositions contain a therapeutically or diagnostically effective dose of the Her2 binding protein of the invention. The amount of protein to be administered depends on the organism to be treated, the type of disease, the age and weight of the patient and further factors known *per se.*

Some embodiments of the invention describe Her2 binding proteins that bind with high affinity of at least 700 nM to the extracellular domain of Her2 but have no or only weak cellular binding. Such Her2 binding proteins are particularly useful for certain medical applications requiring a differentiation of Her2-binding proteins between soluble and cell-bound receptor. Soluble Her2 is often found in the blood of cancer patients. The Her2 binding proteins that bind to soluble Her2 (as for example in Biacore assays) but not to cell-bound receptors can be used for diagnostic applications where soluble Her2 is a predictive biomarker for disease progression. Further, certain therapeutic applications for Her2-binding Affilin proteins that only bind to soluble Her2 can be useful, in particular in combination with a therapeutic antibody that binds soluble and cell bound receptor receptor (e.g. Trastuzumab). In this case, the Affilin would preferably bind the soluble Her2 molecules, leaving more antibody molecules available for the therapeutic intervention at the cell. This opens the opportunity to lower the dose of the antibody known for its cardiotoxic side effects. Examples of such Her2-binding proteins are provided in this invention (e.g. Affilin-142465, Affilin-142655, Affilin-142502, Affilin-141965, and Affilin-144567).

The invention covers a pharmaceutical composition comprising the Her2 binding protein, fusion protein or conjugate or the nucleic acid molecule of the invention, the vector of the invention, and/or the host cell or a virus and a pharmaceutically acceptable carrier. The invention further covers a diagnostic agent comprising the Her2 binding protein or conjugate or the nucleic acid molecule of the invention, the vector of the invention, and/or the host cell or non-human host with a diagnostically acceptable carrier. The compositions contain a pharmaceutically or diagnostically acceptable carrier and optionally can contain further auxiliary agents and excipients known *per se.* These include for example but are not limited to stabilizing agents, surface-active agents, salts, buffers, coloring agents etc.

The pharmaceutical composition comprising the Her2 binding protein can be in the form of a liquid preparation, a lyophilisate, a cream, a lotion for topical application, an aerosol, in the form of powders, granules, in the form of an emulsion or a liposomal preparation. The compositions are preferably sterile, non-pyrogenic and isotonic and contain the pharmaceutically conventional and acceptable additives known *per se.* In addition, reference is made to the regulations of the U.S. Pharmacopoeia or Remington's Pharmaceutical Sciences, Mac Publishing Company (1990).

In the field of human and veterinary medical therapy and prophylaxis pharmaceutically effective medicaments containing at least one Her2 binding protein in accordance with the invention can be prepared by methods known *per se.* Depending on the galenic preparation these compositions can be administered parentally by injection or infusion, systemically, intraperitoneally, intramuscularly, subcutaneously, transdermally or by other conventionally employed methods of application. The type of pharmaceutical preparation depends on the type of disease to be treated, the route of administration, the severity of the disease, the patient to be treated and other factors known to those skilled in the art of medicine.

In a still further aspect the invention discloses diagnostic compositions comprising Her2 binding protein according to the invention specifically binding specific targets/antigens or its isoforms together with diagnostically acceptable carriers. Since enhanced Her2 expression is correlated with tumor malignancy, it is desirable to develop diagnostics for non-invasive imaging in order to gain information about Her2 expression status in patients. Furthermore, Her2 imaging could be useful for the assessment of the response of a patient to a therapeutic treatment. For example, using a Her2 binding protein of the invention labelled with a suitable radioisotope or fluorophore can be used for non-invasive imaging to determine the location of tumors and metastasis (for review see for example Milenic et al. 2008 Cancer Biotherapy & Radiopharmaceuticals 23: 619-631; Hoeben et al. 2011, Int. Journal Cancer 129: 870-878). Due to their pharmacokinetic characteristics, intact antibodies are not suitable for routine imaging. Due to their small size and high affinity, radiolabelled or fluorescently labelled fusion proteins of the invention are expected to be much better suited for use as diagnostics for imaging.

It is expected that a Her2 binding protein of the invention can be advantageously applied in therapy. In particular, the molecules are expected to show superior tumor targeting effect and desired biodistribution and thus, reduced side effects. Pharmaceutical compositions of the invention may be manufactured in any conventional manner.

The derivatization of ubiquitin to generate a ubiquitin mutein that specifically binds to a particular target antigen has been described in the art. For example, a library can be created in which the sequence as shown in SEQ ID NO: 4 has been altered. Preferably, the alterations comprise at least 12 amino acids selected from positions R42, 144, H68, V70, R72, L73, R74, K82, L84, Q138, K139, E140, S141, and T142 of di-ubiquitin (SEQ ID NO: 4). In other embodiments, a library can be created in which the sequence as shown in SEQ ID NO: 1 has been altered at least at amino acids located in positions 62, 63, 64, 65, 66 of SEQ ID NO: 1 in combination with an extension of 4-10 amino acids in the N-terminal loop. Additional 1, 2, 3, 4, 5, or 6 amino acids can be substituted to generate a protein with a novel binding ability to Her2.

The step of modification of the selected amino acids is performed preferably on the genetic level by random mutagenesis of the selected amino acids. Preferably, the modification of ubiquitin is carried out by means of methods of genetic engineering for the alteration of a DNA belonging to the respective protein.

Preferably, the alteration is a substitution, insertion or deletion as described in the art. The substitution of amino acid residues for the generation of the novel binding proteins derived from ubiquitin can be performed with any desired amino acid. This is described in detail in EP1626985B1, EP2379581B1, and EP2721152.

The substitution of amino acids for the generation of the novel binding proteins based on SEQ ID NO: 4 or SEQ ID NO: 1 can be performed with any desired amino acid. This is described in detail for example in EP1626985B1 and EP2379581B1. Assuming a random distribution of the 20 natural amino acids at e.g. 14 positions generates a pool of 20 to the power of 14 (20¹⁴) theoretical ubiquitin muteins, each with a different amino acid composition and potentially different binding properties. This large pool of genes constitutes a library of different Affilin binding proteins.

By way of example, starting point for the mutagenesis can be for example the cDNA or genomic DNA coding for proteins of SEQ ID NOs: 4 and 1. Furthermore, the gene coding for the protein as shown in SEQ ID NOs: 4 and 1 can also be prepared synthetically. The DNA can be prepared, altered, and amplified by methods known to those skilled in the art. Different procedures known *per se* are available for mutagenesis, such as methods for site-specific mutagenesis, methods for random mutagenesis, mutagenesis using PCR or similar methods. All methods are known to those skilled in the art.

The amino acid positions to be mutagenized may be predetermined. In each case, a library of different mutants is generally established using methods known *per se.* Generally, a pre-selection of the amino acids to be modified can be performed based on structural information available for the ubiquitin protein to be modified. The selection of different sets of amino acids to be randomized leads to different libraries.

The gene pool libraries obtained as described above can be combined with appropriate functional genetic elements which enable expression of proteins for selection methods such as display methods. The expressed proteins are contacted with a target molecule to enable binding of the partners to each other if a binding affinity exists. This process enables identification of those proteins which have a binding activity to the target molecule. See, for example, EP2379581B1.

Contacting is preferably performed by means of a suitable presentation and selection method such as the phage display, ribosomal display, mRNA display or cell surface display, yeast surface display or bacterial surface display methods, preferably by means of the phage display method. For complete disclosure, reference is made also to the following references: Hoess, Curr. Opin. Struct. Biol. 3 (1993), 572-579; Wells and Lowmann, Curr. Opin. Struct. Biol. 2 (1992), 597-604; Kay et al., Phage Display of Peptides and Proteins-A Laboratory Manual (1996), Academic Press. The methods mentioned above are known to those skilled in the art.

The library can be cloned into a phagemid vector (e.g. pCD87SA (Paschke, M. and W. Hohne (2005). " Gene 350(1): 79-88)). The library may be displayed on phage and subjected to repeated rounds of panning against the respective target antigen. Ubiquitin muteins from enriched phage pools are cloned into expression vectors for individual protein expression. Preferably, expression of the ubiquitin mutein enables screening for specific binding proteins by established techniques, such as ELISA on automated high-throughput screening platforms. Identified clones with desired binding properties are then sequenced to reveal the amino acid sequences of Affilin molecules. The identified binding protein may be subjected to further maturation steps, e.g. by generating additional libraries based on alterations of the identified sequences and repeated phage display, ribosomal display, panning and screening steps as described above.

Her2 binding molecules of the invention may be prepared by any of the many conventional and well known techniques such as plain organic synthetic strategies, solid phase-assisted synthesis techniques, fragment ligation techniques or by commercially available automated synthesizers. On the other hand, they may also be prepared by conventional recombinant techniques alone or in combination with conventional synthetic techniques. Furthermore, they may also be prepared by cell-free in-vitro transcription/translation. Conjugates according to the present invention may be obtained by combining compounds by chemical methods, e.g. lysine or cysteine-based chemistry, as described herein above.

According to another aspect of the invention, an isolated polynucleotide encoding a Her2 binding protein of the invention is provided. The invention also encompasses polypeptides encoded by the polynucleotides of the invention. The invention further provides an expression vector comprising the isolated polynucleotide of the invention, and a host cell comprising the isolated polynucleotide or the expression vector of the invention.

For example, one or more polynucleotides which encode for a Her2 binding protein of the invention may be expressed in a suitable host and the produced binding protein can be isolated. Vectors comprising said polynucleotides are covered by the invention. In a further embodiment the invention relates to a vector comprising the nucleic acid molecule of the invention. A vector means any molecule or entity (e.g., nucleic acid, plasmid, bacteriophage or virus) that can be used to transfer protein coding information into a host cell.

The present invention furthermore relates to an isolated cell comprising the nucleic acid molecule of the invention or the vector of the invention. Suitable host cells include prokaryotes or eukaryotes. Various mammalian or insect cell culture systems can also be employed to express recombinant proteins.

The invention also relates in an embodiment to a host cell or a non-human host carrying the vector of the invention. A host cell is a cell that has been transformed, or is capable of being transformed, with a nucleic acid sequence and thereby expresses a gene of interest. The term includes the progeny of the parent cell, whether or not the progeny is identical in morphology or in genetic make-up to the original parent cell, so long as the gene of interest is present.

In accordance with the present invention, the host may be a transgenic non-human animal transfected with and/or expressing the Her2 binding proteins of the present invention. In a preferred embodiment, the transgenic animal is a non-human mammal.

In another aspect, there is provided a method of producing the Her2 binding protein of the invention, comprising the steps of a) culturing the host cell of the invention under conditions suitable for the expression of the Her2 binding protein and b) isolating the produced Her2 binding protein. The invention also encompasses a Her2 binding protein produced by the method of the invention. Suitable conditions for culturing a prokaryotic or eukaryotic host are well known to the person skilled in the art.

One embodiment of the present invention is directed to a method for the preparation of a Her2 binding protein according to the invention as detailed above, said method comprising the following steps: (a) preparing a nucleic acid encoding a Her2 binding protein according to any aspect of the invention; (b) introducing said nucleic acid into an expression vector; (c) introducing said expression vector into a host cell; (d) cultivating the host cell; (e) subjecting the host cell to culturing conditions under which a Her2 binding protein is expressed, thereby producing a Her2 binding protein as described above; (f) optionally isolating the Her2 binding protein produced in step (e); and (g) optionally conjugating the Her2 binding protein with further functional moieties as described above.

Cultivation of cells and protein expression for the purpose of protein production can be performed at any scale, starting from small volume shaker flasks to large fermenters, applying technologies well-known to any skilled in the art.

Following the expression of the Her2 binding protein of the invention, it can be further purified and enriched by methods known *per se.* The selected methods depend on several factors known *per se* to those skilled in the art, for example the expression vector used, the host organism, the intended field of use, the size of the protein and other factors.

In general, isolation of purified protein from the cultivation mixture can be performed applying conventional methods and technologies well known in the art, such as centrifugation, precipitation, flocculation, different embodiments of chromatography, filtration, dialysis, concentration and combinations thereof, and others. Chromatographic methods are well-known in the art and comprise without limitation ion exchange chromatography, gel filtration chromatography (size exclusion chromatography), or affinity chromatography.

For simplified purification the Her2 binding protein of the invention can be fused to other peptide sequences having an increased affinity to separation materials. Preferably, such fusions are selected that do not have a detrimental effect on the functionality of the ubiquitin mutein or can be separated after the purification due to the introduction of specific protease cleavage sites. Such methods are also known to those skilled in the art.

### EXAMPLES

The following Examples are provided for further illustration of the invention. The invention is particularly exemplified by particular modifications of di-ubiquitin (SEQ ID NOs: 4 or 48) or wild type ubiquitin (SEQ ID NOs: 1 or 2) resulting in binding to Her2. The invention, however, is not limited thereto, and the following Examples merely show the practicability of the invention on the basis of the above description. For a complete disclosure of the invention reference is made also to the literature cited in the specification.

### Example 1. Identification of binding proteins

*Library Construction and Cloning* Two ubiquitin moieties each comprising seven randomized amino acid positions were synthesized by triplet technology (MorphoSys Slonomics, Germany) to achieve a well-balanced amino acid distribution. A mixture of 19-amino acid coding premade double-stranded triplets excluding cysteine was used for the synthesis. Both ubiquitin moieties were directly linked (without linker between the two ubiquitin moieties) in head to tail orientation to result in a protein of 152 amino acids with 14 randomized amino acid positions. The sequence of di-ubiquitin with 14 randomized positions is shown in SEQ ID NO: 3:

The 14 randomized amino acids correspond to positions 42, 44, 68, 70, 72, 73, 74, 82, 84, 138, 139, 140, 141, and 142 of di-ubiquitin. The sequence of di-ubiquitin is shown in SEQ ID NO: 4:

The construct was ligated with a modified pCD87SA phagemid (herein referred to as pCD12) using standard methods known to a skilled person. The pCD12 phagemid comprises a modified *torA* leader sequence (deletion of amino acid sequence QPAMA) to achieve protein processing without additional amino acids at the N terminus. Aliquots of the ligation mixture were used for electroporation of *Escherichia coli* ER2738 (Lucigen).

The library is referred to as SPIF. Unless otherwise indicated, established recombinant genetic methods were used, for example as described in Sambrook *et al.*

*Target:* Recombinant human Her2 -Fc Chimera was purchased from R&D Systems. A DNA sequence encoding the extracellular domain of human Her2 (uniprot Accession Number p004626; residues 1-652) was genetically fused with the Fc region of human IgG1 at the C-terminus.

*TAT Phage Display Selection.* The SPIF library was enriched against the given protein target Her2 using TAT phage display as selection system. After transformation of competent bacterial ER2738 cells (Lucigene) with phagemid pCD12 carrying the SPIF library, phage amplification and purification was carried out using standard methods known to a skilled person. For selection the target protein was provided as Fc-fusion protein (Her2-Fc, R&D Systems) immobilized on Dynabeads® Protein A or G. The target concentration during phage incubation varied from 200 nM (first round) to 50 nM (third round). Target phage complexes were magnetically separated from solution and washed several times. Target bound phages were eluted by trypsin. To deplete the phage library for Fc-binding variants a preselection of phages with immobilized Fc-fragment of IgG1 (Athens Research & Technology) was performed prior to round two and three.

To identify target specific phage pools, eluted and reamplified phages of each selection round were analysed by phage pool ELISA. Wells of a medium binding microtiter plate (Greiner bio-one) were coated with Her2-Fc (2,5 µg/ml) and Fc-fragment of IgG1 (5 µg/ml), respectively. Bound phages were detected using α-M13 HRP-conjugated antibody (GE Healthcare). Eluted and reamplified phages of round three showed specific binding to the target and were used subsequently for pool maturation by error prone PCR. Isolated phagemid pools served as template for error prone PCR (GeneMorph II Random Mutagenesis Kit, Agilent Technologies). The amplified pool of SPIF variants carrying now additional substitutions compared to the library positions was recloned into phagemid pCD12 and transformed into ER2738 for phage amplification und purification. The phages were again subjected to two rounds of panning as described above. The target was employed at a concentration of 5 nM and 1 nM in round one and two, respectively. For both rounds a preselection with Fc-fragment of IgG1 was performed. To analyze the matured and selected pools for specific target binding a phage pool ELISA was performed as described above.

*Cloning of Target Binding Phage Pools into an Expression Vector.* Upon completion of the selection procedure the target specific DNA pools of maturation selection round one and two were amplified by PCR according to methods known in the art, cut with appropriate restriction nucleases and ligated into a derivative of the expression vector pET-28a (Merck, Germany) comprising a Strep-Tag II (IBA GmbH).

*Single Colony Hit Analysis.* After transformation of BL21 (DE3) cells (Merck, Germany) kanamycin-resistant single colonies were grown. Expression of the target-binding modified ubiquitin variants was achieved by cultivation in 384 well plates (Greiner BioOne) using auto induction medium (Studier, 2005). Cells were harvested and subsequently lysed chemically or enzymatically by BugBuster reagent (Novagen) or mechanically by freeze/thaw cycles, respectively. After centrifugation the resulting supernatants were screened by ELISA with immobilized target on highbind 384 micrrotiter plates (Greiner BioOne). Detection of bound protein was achieved by *Strep*-Tactin® HRP Conjugate (IBA GmbH) in combination with TMB-Plus substrate (Biotrend, Germany). The reaction was stopped by addition of 0.2 M H₂SO₄ solution and measured in a plate reader at 450 nm versus 620 nm.

### Example 2. Expression and purification of Her2-binding proteins

Affilin molecules were cloned to an expression vector using standard methods known to a skilled person, purified and analyzed as described below. All Affilin proteins were expressed and highly purified by affinity chromatography and gel filtration. After affinity chromatography purification a size exclusion chromatography (SE HPLC or SEC) has been performed using an Äkta system and a Superdex™ 200 HiLoad 16/600 column (GE Healthcare). The column has a volume of 120 ml and was equilibrated with 2 CV. The samples were applied with a flow rate of 1 ml/min purification buffer B. Fraction collection starts as the signal intensity reaches 10 mAU. Following SDS-PAGE analysis positive fractions were pooled and their protein concentrations were measured.

Further analysis included SDS-PAGE, SE-HPLC and RP-HPLC. Protein concentrations were determined by absorbance measurement at 280 nm using the molar absorbent coefficient. RP chromatography (RP HPLC) has been performed using a Dionex HPLC system and a Vydac 214MS54 C4 (4.6 x 250 mm, 5µm, 300 Å) column (GE Healthcare).

### Example 3. Solubility analysis of Her2 binding proteins

Supernatants and resuspended pellets were analyzed by NuPage Novex 4-12 % Bis-Tris SDS gels and stained with Coomassie. Proteins were recovered from the pellets by addition of 8 M urea. Her2 binding proteins displayed a high solubility of at least 80 % soluble (SEQ ID NO: 5, 27, 30, 37, 38), at least 90 % soluble (SEQ ID NOs: 6, 20, 23, 28, 34), at least 95 % soluble expression (SEQ ID NOs: 7, 9, 10, 11, 22, 29), 100 % soluble (SEQ ID NOs: 8, 12, 13, 14, 15, 16, 17, 18, 19, 21, 25, 26, 33, 35, 36).

### Example 4. Her2 binding proteins are stable at high temperatures

Thermal stability of the binding proteins of the invention was determined by Differential Scanning Fluorimetry (DSF). Each probe was transferred at concentrations of 0.1 µg/µL to a MicroAmp Optical 384-well plate well plate, and SYPRO Orange dye was added at suitable dilution. A temperature ramp from 25 to 95 °C was programmed with a heating rate of 1 °C per minute (ViiA-7 Applied Biosystems). Fluorescence was constantly measured at an excitation wavelength of 520 nm and the emission wavelength at 623 nm (ViiA-7, Applied Biosystems). The midpoints of transition for the thermal unfolding (Tm, melting points) are shown for selected variants in Figure 2. Her2 binding proteins of the invention have similar melting temperatures. The stability of all binding proteins is comparable to the stability of the control proteins.

### Example 5. Analysis of Her2 binding proteins (Surface Plasmon Resonance, SPR)

A CM5 sensor chip (GE Healthcare) was equilibrated with SPR running buffer. Surface-exposed carboxylic groups were activated by passing a mixture of EDC and NHS to yield reactive ester groups. 700-1500 RU Her2-Fc(*on*-ligand) were immobilized on a flow cell, IgG-Fc (*off-*ligand) was immobilized on another flow cell at a ratio of 1:3 (hIgG-Fc:Target) to the target. Injection of ethanolamine after ligand immobilization was used to block unreacted NHS groups. Upon ligand binding, protein analyte was accumulated on the surface increasing the refractive index. This change in the refractive index was measured in real time and plotted as response or resonance units (RU) versus time. The analytes were applied to the chip in serial dilutions with a flow rate of 30 µl/min. The association was performed for 30 seconds and the dissociation for 60 seconds. After each run, the chip surface was regenerated with 30 µl regeneration buffer and equilibrated with running buffer. A dilution series of Trastuzumab served as positive control, whereas a dilution series of unmodified di-ubiquitin represents the negative control. The control samples were applied to the matrix with a flow rate of 30 µl/min, while they associate for 60 seconds and dissociate for 120 seconds. Regeneration and re-equilibration were performed as previously mentioned. Binding studies were carried out by the use of the B iacore 3000 (GE Healthcare); data evaluation was operated via the BIAevaluation 3.0 software, provided by the manufacturer, by the use of the Langmuir 1:1 model (RI=0). Results of binding to Her2 are shown in Figure 2. Evaluated dissociation constants (K_{D}) were standardized against *off*-target and indicated.

### Example 6. Functional characterization: Binding to cell surface expressed Her2 (Flow Cytometry)

Flow cytometry was used to analyze the interaction of Her2 binding proteins with surface-exposed Her2. Her2 overexpressing human mammary gland adenocarcinoma-derived SkBr3 cells, Her2 overexpressing transfected CHO-K1 cells (chinese hamster ovary cells), Her2 non-expressing human embryonic kidney cell line HEK/293 and empty vector control CHO-K1 cells were used. Results are summarized in Figures 4 to 8.

Cells were trypsinized and resupended in medium containing FCS, washed and stained in pre-cooled FACS blocking buffer. A cell concentration of 2x10⁶ cells/ml was prepared for cell staining and filled into a 96 well plate (Greiner) in triplicate for each cell line.

Different concentrations of Affilin proteins were added to Her2 overexpressing and control cells in several experiments. 50 nM of each Affilin was tested on SkBr3 and Her2-negative HEK/293-cells (Figure 4A and 4B). A dilution series from 333 nM to 5.6 nM (Figure 5) and a dilution series from 100 nM to 0.06 pM (Figure 8) were added to SkBr3-cells. On Her2-overexpressing CHO-K1 cells and the Her2-negative CHO-K1-pEntry cell line Affilin concentrations of 500 nM to 0.5 nM (Figure 6 and Figure 7) were tested. After 45 min the supernatants were removed and 100 µl/well rabbit anti-Strep-Tag antibody (obtained from GenScript; A00626), 1:300 diluted in FACS blocking buffer were added. After removal of the primary antibody goat anti-rabbit IgG Alexa Fluor 488 antibody (obtained from Invitrogen; A11008) was applied in a 1:1000 dilution. Flow cytometry measurement was conducted on the Guava easyCyte 5HT device from Merck-Millipore at excitation wavelength 488 nm and emission wavelength 525/30 nm. Results on SkBr3 for binding of Affilin-141884, Affilin-141890, Affilin-141912, Affilin-141926, Affilin-141931, Affilin-141935, Affilin-141965, Affilin-141975 (Figure 4A), Affilin-142418, Affilin-142437, Affilin-142465, Affilin-142502, Affilin-142609, Affilin-142618, Affilin-142620, Affilin-142627, Affilin-142628, Affilin-142654, Affilin-142655, Affilin-142672, Affilin-141884 (Figure 4B) and Affilin-141926 and Affilin-141890 (Figure 5) are shown. Results on CHO-K1-Her2 cells for binding of Affilin-142628, Affilin-142654, Affilin-141884, Affilin-142627, Affilin-144631, Affilin-144632, Affilin-144633, Affilin-144634, Affilin-144635, Affilin-144636, Affilin-144637, Affilin-144567, and Affilin-142502 are depicted in Figure 6a-c. Concentration dependent binding of (50 nM to 0.5 nM) Affilin-142628 to CHO-K1-Her2 cells and CHO-K1-pEntry cells is shown in Figure 7. Comparable amounts of di-ubiquitin (139090) were used as negative control in the experiments where applicable (e.g. in experiments as shown in Figures 4A, 4B, 6A, 6B, and 7). However, Affilin-142465 (SEQ ID NO: 33), Affilin-142655 (SEQ ID NO: 34), Affilin-142502 (SEQ ID NO: 49), and Affilin-141965 (SEQ ID NO: 50) showed only weak binding to Her2 overexpressing SkBr3 cells. SEQ ID NOs: 34, 35, 49 and 50 have at least the following substitutions: 42S, 44V, 68Y, 70Y, 72F, 73S, 82L, 84D, 138R, 139G, 140W, 142L (of di-ubiquitin). Affilin-142418 and Affilin-142655 have further two substitutions (K63I, Q78R) or further four substitutions (Q31L, D58V, Q78R, P95S), respectively. Her2 binding proteins that bind with high affinity of at least 700 nM to the extracellular domain of Her2 but without or with low cellular binding are particularly useful for certain applications, e.g. for certain diagnostic or therapeutic applications that require Her-binding proteins with high affinity only for soluble Her2, but not for cellular Her2. Affilin-142418 shows binding to Her2 overexpressing SkBr3 cells (Figure 4b, Figure 9).

### Example 7. Binding to cell surface expressed Her2 (Immunocytochemistry and fluorescence microscopy)

Binding of proteins of the invention on cells exogenously expressing human Her2 was confirmed. 50 nM of Affilin-141884, Affilin-142628, Affilin-141926, Affilin-144637, Affilin-142418 and Affilin-144567 were tested on Her2-expressing SkBr3 cells and the negative control cell line HEK/293. Di-ubiquitin (139090) was used as negative control and 10nM Trastuzumab served as positive control. Cells were seeded with a concentration of 1x10⁵cells/ml in Lab-Tek® Chamber-Slides (Sigma-Aldrich). After cultivation over 72 h the cells were fixed with methanol (5 min., 20 °C), followed by blocking (5 % Fetal Horse Serum in PBS, 1 h) and incubation with 50 nM Affilin for 45 min at rt. Affilin binding was detected by an incubation of rabbit-anti-Strep-Tag-antibody (1:500) for 1h and subsequent incubation with anti-rabbit-lgG-Alexa488-antibody (1:1000) for 1 h. Trastuzumab binding were proved with anti-human-IgG-Alexa488-antibody (1:1000). The nuclei were stained with 4 µg/ml DAPI. All incubation steps were done at room temperature. Figure 9A shows strong binding of Affilin-141884, Affilin-142628, Affilin-141926, Afflin-144637 and Affilin-142418. Weak or negative binding of Affilin-144567 and di-ubiquitin is shown in Figure 9B. Comparable binding was detected with Trastuzumab. No non-specific binding was observed on Her2-negative cell line HEK/293.

### Example 8. Functional characterization: Her2 binding proteins bind to extracellular Her2 expressed on tumor cells (immunohistochemistry and fluorescence microscopy)

Cryo-tissue sections of SKOV-3-tumor, lung, liver, heart muscle and ovary were used to analyze the binding proteins of the invention. Tissue slices were fixed with ice-cold Acetone for 10 min, followed by blocking and incubation with different concentrations (20 nM and 50 nM) of Affilin-141884 and Affilin-142628 and an equal amount of di-ubiquitin clone 139090 as negative or 10 nM Trastuzumab as positive control for 1 h. After washing with PBS the tissue was incubated with rabbit anti-Strep-Tag antibody (1:500) for 1 h at room temperature, followed by an incubation with goat anti-rabbit Alexa488 (1:1000) or goat anti-human IgG Alexa594 (1:1000) as secondary antibody for Trastuzumab. Nuclei of cells were visualized with DAPI. Chamber slides were dissembled and the glass slides were covered with Mowiol and a cover glass. Slices were imaged at a Zeiss Axio Scope.A1 microscope and images were processed using standard software packages. Figure 10 and 11 show the specific binding of Affilin-141884 and Affilin-142628 on SKOV-3-tumor-slices compared to the non-binding protein clone 139090. No binding on lung tissue was obtained (Figure 11). Slices of further tissues (liver, heart muscle and ovarian tissue) were tested; no specific staining was observed.

### Example 9. Competition Analysis that Her2 binding Affilin molecules bind to other epitope than anti-Her2 monoclonal antibody Trastuzumab

Affilin proteins that bind to different Her2 epitopes of particular Trastuzumab can be useful in certain medical embodiments. To investigate whether the isolated Her2 binding proteins of the invention can compete with the anti-Her2 monoclonal antibody Trastuzumab, the following assay was performed: Her2 (from Acrobiosystems) was immobilized on a CM5 Biacore chip using NHS/EDC chemistry resulting in 1000 response units (RU). In a first experiment, all variants were injected at one defined concentration (2.5 µM) at a flow of 30 µl/min PBST 0.005 % Tween 20 (Figure 12). In the second experiment, the same flow channel was first pre-loaded with Trastuzumab (200 nM) until the chip surface was saturated (Figure 13). After loading Trastuzumab, the variants were identically applied as in experiment 1 (2.5 µM). For better clarification both sensogram traces were aligned at the last injected Her2 binding protein.

It was demonstrated that the binding of a Her2 binding protein was not influenced by the presence of Trastuzumab. Thus, no competition was observed, meaning that Trastuzumab and Her2 binding proteins of the invention bind to different or non-overlapping Her2-epitopes, i.e. to different surface exposed amino acids, than Trastuzumab (see Figure 13). This was observed for Affilin-142628 (SEQ ID NO: 19), Affilin-143692 (SEQ ID NO: 39), Affilin-141926 (SEQ ID NO: 28), Affilin-141884 (SEQ ID NO: 38), Affilin-141890 (SEQ ID NO: 30), and Affilin-141975 (SEQ ID NO: 37). These binding proteins might be particularly useful in cancer treatments with reported primary and acquired resistance to Trastuzumab. Affilin-141931 (SEQ ID NO: 27), Affilin-141912 (SEQ ID NO: 31), and Affilin-141935 (SEQ ID NO: 32) bind to identical or overlapping Her2-epitopes as Trastuzumab.

### Example 10: Binding analysis of bispecific fusion proteins of Her2 binding protein and EGFR-antibody Cetuximab

A Her2 binding protein was linked to the C- or N-terminus of the light chain or heavy chain of the anti-EGFR monoclonal antibody Cetuximab. Fusion proteins were generated by fusing a Her2 binding protein (for example, Affilin-141926) to the N-terminus of the heavy chain of the anti EGFR antibody Cetuximab (referred to as NH-141926; SEQ ID NO: 47), to the C- terminus of the heavy chain of the anti EGFR antibody Cetuximab (referred to as CH-141926; SEQ ID NO: 45), to the N-terminus of the light chain of the anti EGFR antibody Cetuximab (referred to as NL-141926; SEQ ID NO: 46), and to the C- terminus of the light chain of the anti-EGFR antibody Cetuximab (Erbitux®; referred to as CL-141926; SEQ ID NO: 44) respectively. The first up to 20 amino acids of SEQ ID NOs: 44-47 are signal sequences. The cDNA encoding the fusion proteins were transiently transfected into FreeStyle™ 293-F cells and expressed in serum-free/animal component-free media. Expression was confirmed by Western Blot analysis. Fusion proteins were purified from the supernatants by Protein A affinity chromatography (GE-Healthcare cat no 17-0402-01) with an ÄKTAxpress® (GE Healthcare). Further purification of the fusion proteins was achieved by gel filtration. Further analysis included SDS-PAGE, SE-HPLC and RP-HPLC. Thermal stability of the binding proteins of the invention was determined by Differential Scanning Fluorimetry as described above. The midpoint of transition for the thermal unfolding (Tm, melting points) was determined for fusion proteins; all fusions proteins have thermal stabilities between Tₘ= 63.9°C and 67.9°C (see Tab. 3). The stability of all binding proteins is comparable to the stability of the control proteins.

**Tab. 3: Midpoint of transition for the thermal unfolding of binding proteins of the invention and of control proteins**

| **Fusion protein or control** | **Tm [°C]** |
|---|---|
| Cetuximab | 69.0 |
| CH-141926 | 67.4 |
| CL-141926 | 63.9 |
| NH-141926 | 67.5 |
| NL-141926 | 67.9 |

Binding studies were carried out by the use of the Biacore® 3000 (GE Healthcare) as described above and as shown in Figure 14. Further, FACS analysis of binding of the fusion proteins to the target receptors expressed individually in CHO-K1 cells confirmed cell binding (Figure 15). Results are summarized in Tab. 4 and Tab. 5.

**Tab. 4: Affinity data for Her2-ubiquitin-mutein-Cetuximab binding proteins of the invention for EGFR (Biacore)**

| **Fusion protein** | **kₒₙ [M⁻¹ x s⁻¹]** | **k_{off} [s⁻¹]** | **K_{D} [M]** |
|---|---|---|---|
| Cetuximab | 6.21 x 10⁵ | 6.29 x 10⁻⁴ | 1.01 x 10⁻⁹ |
| CH-ubiquitin | 7.28 x 10⁵ | 6.72 x 10⁻⁴ | 9.23 x 10⁻¹⁰ |
| CH-141926 | 4.66 x 10⁵ | 1.65 x 10⁻⁴ | 3.53 x 10⁻¹⁰ |
| CL-ubiquitin | 7.96 x 10⁵ | 7.12 x 10⁻⁴ | 8.95 x 10⁻¹⁰ |
| CL-141926 | 5.84 x 10⁵ | 5.91 x 10⁻⁴ | 1.01 x 10⁻⁹ |
| NH-ubiquitin | 3.02 x 10⁵ | 6.5 x 10⁻⁴ | 2.15 x 10⁻⁹ |
| NH-141926 | 3.79 x 10⁵ | 4.12 x 10⁻⁴ | 1.09 x 10⁻⁹ |
| NL-ubiquitin | 2.79 x 10⁵ | 1.54 x 10⁻⁵ | 5.52 x 10⁻¹¹ |
| NL-141926 | 1.08 x 10⁵ | 1.72 x 10⁻⁴ | 1.59 x 10⁻⁹ |

**Tab. 5: Affinity data for Her2-ubiquitin-mutein-Cetuximab binding proteins of the invention for Her2 (Biacore)**

| **Fusion protein** | **kₒₙ [M⁻¹ x s⁻¹]** | **k_{off} [s⁻¹]** | **K_{D} [M]** |
|---|---|---|---|
| CH-141926 | 7.53 x 10⁴ | 4.96 x 10⁻⁴ | 6.58 x 10⁻⁹ |
| CL-141926 | 3.11 x 10⁵ | 4.46 x 10⁻⁴ | 1.43 x 10⁻⁹ |
| NH-141926 | 9.03 x 10⁴ | 3.05 x 10⁻⁴ | 3.37 x 10⁻⁹ |
| NL-141926 | 8.23 x 10⁴ | 2.36 x 10⁻⁴ | 2.87 x 10⁻⁹ |

It is known that a co-expression of the receptor proteins EGFR and Her2 on various forms of cancer (e.g. breast, colorectal and prostate cancer) is associated with poor prognosis for the patients. In Figure 14, a Biacore chip with immobilized extracellular domain Her2-Fc was used. The bispecific fusion proteins were injected, after 350 sec, extracellular domain EGFR-Fc was injected at concentrations between 100 nM and 25 nM in 1:2 dilution series.

Figure 14 shows the simultaneous binding of bispecific Affilin-antibody fusion proteins to both targets. This effect might increase the selectivity and efficiency of a product comprising a bispecific EGFR-Her2-fusion protein of the invention in therapeutic applications in molecular imaging. In Figure 15 the binding of the two interaction moieties to their respective targets is shown on Her2 (Figure 15a) and EGFR (Figure 15b) overexpressing CHO cells by flow cytometry measurements for variant CL 141926.

## Claims

1. A Her2 binding protein comprising an amino acid sequence wherein 12, 13, or 14 amino acids selected from positions R42, 144, H68, V70, R72, L73, R74, K82, L84, Q138, K139, E140, S141, and T142 of di-ubiquitin having the amino acid sequence of SEQ ID NO: 4 are substituted, and wherein the Her2 binding protein has at least 85% sequence identity to di-ubiquitin of SEQ ID NO: 4, and wherein the Her2 binding protein has a binding affinity (K_{D}) of less than 700 nM for Her2, wherein the amino acid at
position 42 is substituted by a polar amino acid,
position 44 is substituted by a hydrophobic or polar amino acid,
position 68 is substituted by an aromatic amino acid,
position 70 is substituted by an aromatic amino acid,
position 72 is substituted by a polar or aromatic amino acid,
position 73 is substituted by any amino acid but not basic or acidic amino acid,
position 74 is substituted by an aromatic, basic or polar amino acid,
position 82 is substituted by any amino acid but not basic or acidic amino acid,
position 84 is substituted by a basic or acidic amino acid,
position 138 is substituted by a basic or acidic or polar amino acid,
position 139 is substituted by an acidic or hydrophobic amino acid or glycine,
position 140 is substituted by an aromatic amino acid,
position 141 is substituted by a hydrophobic or polar or basic amino acid, and/or position 142 is substituted by a hydrophobic or polar amino acid.

2. The Her2 binding protein according to claim 1, comprising 1, 2, 3, 4, 5, or 6 further substitutions of di-ubiquitin.

3. The Her2 binding protein according to claim 1, wherein the amino acids are selected from
R42T, R42S, R42L,
I44A, I44V, I44S, I44T,
H68W, H68Y, H68F,
V70Y, V70W,
R72T, R72F, R72G, R72Y,
L73W, L73S, L73V, L73I,
R74Y, R74S, R74N, R74K,
K82T, K82L, K82N, K82I, K82Y,
L84H, L84D, L84E, L84S,
Q138S, Q138R, Q138E,
K139E, K139G, K139L,
E140W,
S141A,
S141R,
T142I, T142L, and/or T142N.

4. The Her2 binding protein according to claim 3, wherein the amino acids are selected from
Q138S, K139E, E140W, S141A, and T142I; or
Q138R, K139G, E140W, and T142L; or
Q138E, K139L, E140W, S141R, and T142N.

5. The Her2 binding protein according to claim 3, wherein the amino acids are selected from R42T, I44A, H68W, V70Y, R72T, L73W, R74Y, K82T, and L84H.

6. The Her2 binding protein according to claim 3, wherein the amino acids are selected from R42S, I44V, H68Y, V70Y, R72F, L73S, K82L, and L84D.

7. The Her2 binding protein according to any of the preceding claims, wherein the amino acid sequence is selected from one of the amino acid sequences of SEQ ID NOs: 5-38.

8. The Her2 binding protein according to claim 3, wherein the Her2 binding protein binds to a different or non-overlapping Her2 epitope than the monoclonal antibody Trastuzumab.

9. The Her2 binding protein according to any of the preceding claims, further comprising at least one additional molecule, preferably selected from at least one member of the groups (i), (ii), and (iii) consisting of
(i) a moiety modulating pharmacokinetics selected from a polyethylene glycol, a human serum albumin, an albumin-binding peptide, or an immunoglobulin or immunoglobulin fragments, a polysaccharide, and,
(ii) a therapeutically active component, optionally selected from a monoclonal antibody or a fragment thereof, a cytokine, a chemokine, a cytotoxic compound, an enzyme, or derivatives thereof, or a radionuclide, and
(iii) a diagnostic component, optionally selected from a fluorescent compound, a photosensitizer, a tag, an enzyme, or a radionuclide.

10. The Her2 binding protein according to claim 9, comprising a monoclonal antibody with specificity for EGFR.

11. The Her2 binding protein according to any of the preceding claims for use in diagnostics or medicine, preferably for use in the diagnosis or treatment of cancer.

12. A nucleic acid molecule encoding a Her2 binding protein as defined in any one of claims 1 to 11.

13. A vector comprising the nucleic acid molecule of claim 12.

14. A host cell or a non-human host comprising the Her2 binding protein as defined in any one of claims 1 to 11, a nucleic acid as defined in claim 12, and/or a vector of claim 13.

15. A composition comprising the Her2 binding protein as defined in any one of claims 1 to 11, a nucleic acid molecule as defined in claim 12; the vector as defined in claim 13; and/or the host cell as defined claim 14.

16. A method for the production of a Her2 binding protein of any of claims 1 to 11, comprising culturing of the host cell of claim 14 under suitable conditions in order to obtain said Her2 binding protein, and optionally isolating said Her2 binding protein.

## Patentansprüche

1. Her2-bindendes Protein, umfassend eine Aminosäuresequenz, wobei 12, 13 oder 14 Aminosäuren ausgewählt aus den Positionen R42, I44, H68, V70, R72, L73, R74, K82, L84, Q138, K139, E140, S141 und T142 von Di-Ubiquitin mit der Aminosäuresequenz von SEQ ID NO: 4 substituiert sind, und wobei das Her2-bindende Protein mindestens 85% Sequenzidentität zu Di-Ubiquitin von SEQ ID NO: 4 aufweist, und wobei das Her2-bindende Protein eine Bindungsaffinität (K_{D}) von weniger als 700 nM für Her2 aufweist, wobei die Aminosäure an
Position 42 durch eine polare Aminosäure substituiert ist,
Position 44 durch eine hydrophobe oder polare Aminosäure substituiert ist,
Position 68 durch eine aromatische Aminosäure substituiert ist,
Position 70 durch eine aromatische Aminosäure substituiert ist,
Position 72 durch eine polare oder aromatische Aminosäure substituiert ist,
Position 73 durch eine beliebige Aminosäure substituiert ist, jedoch nicht durch eine basische oder saure Aminosäure,
Position 74 durch eine aromatische, basische oder polare Aminosäure substituiert ist,
Position 82 durch eine beliebige Aminosäure substituiert ist, jedoch nicht durch eine basische oder saure Aminosäure,
Position 84 durch eine basische oder saure Aminosäure substituiert ist,
Position 138 durch eine basische oder saure oder polare Aminosäure substituiert ist,
Position 139 durch eine saure oder hydrophobe Aminosäure oder Glycin substituiert ist,
Position 140 durch eine aromatische Aminosäure substituiert ist,
Position 141 durch eine hydrophobe oder polare oder basische Aminosäure substituiert ist, und/oder
Position 142 durch eine hydrophobe oder polare Aminosäure substituiert ist.

2. Das Her2-bindende Protein nach Anspruch 1, umfassend 1, 2, 3, 4, 5 oder 6 weitere Substitutionen von Di-Ubiquitin.

3. Das Her2-bindende Protein nach Anspruch 1, wobei die Aminosäuren ausgewählt sind aus
R42T, R42S, R42L,
I44A, I44V, I44S, I44T,
H68W, H68Y, H68F,
V70Y, V70W,
R72T, R72F, R72G, R72Y,
L73W, L73S, L73V, L73I,
R74Y, R74S, R74N, R74K,
K82T, K82L, K82N, K82I, K82Y,
L84H, L84D, L84E, L84S,
Q138S, Q138R, Q138E,
K139E, K139G, K139L,
E140W,
S141A,
S141R,
T142I, T142L, und/oder T142N.

4. Das Her2-bindende Protein nach Anspruch 3, wobei die Aminosäuren ausgewählt sind aus
Q138S, K139E, E140W, S141A und T142I; oder
Q138R, K139G, E140W und T142L; oder
Q138E, K139L, E140W, S141R und T142N.

5. Das Her2-bindende Protein nach Anspruch 3, wobei die Aminosäuren ausgewählt sind aus R42T, I44A, H68W, V70Y, R72T, L73W, R74Y, K82T und L84H.

6. Das Her2-bindende Protein nach Anspruch 3, wobei die Aminosäuren ausgewählt sind aus R42S, I44V, H68Y, V70Y, R72F, L73S, K82L und L84D.

7. Das Her2-bindende Protein nach einem der vorhergehenden Ansprüche, wobei die Aminosäuresequenz ausgewählt ist aus einer der Aminosäuresequenzen von SEQ ID NOs: 5-38.

8. Das Her2-bindende Protein nach Anspruch 3, wobei das Her2-bindende Protein an ein anderes oder nicht-überlappendes Her2-Epitop als der monoklonale Antikörper Trastuzumab bindet.

9. Das Her2-bindende Protein nach einem der vorhergehenden Ansprüche, weiterhin umfassend mindestens ein zusätzliches Molekül, vorzugsweise ausgewählt aus mindestens einem Mitglied der Gruppen (i), (ii) und (iii) bestehend aus
(i) einem die Pharmakokinetik modulierenden Teil ausgewählt aus einem Polyethylenglykol, einem humanen Serumalbumin, einem Albuminbindenden Peptid, oder einem Immunglobulin oder Immunglobulin-Fragmenten, einem Polysaccharid, und,
(ii) einer therapeutisch wirksamen Komponente, gegebenenfalls ausgewählt aus einem monoklonalen Antikörper oder ein Fragment davon, einem Zytokin, einem Chemokin, einer zytotoxischen Verbindung, einem Enzym, oder Derivaten davon, oder einem Radionuklid, und
(iii) einer diagnostischen Komponente, gegebenenfalls ausgewählt aus einer fluoreszierenden Verbindung, einem Photosensibilisator, einem Tag, einem Enzym, oder einem Radionuklid.

10. Das Her2-bindende Protein nach Anspruch 9, umfassend einen monoklonalen Antikörper mit Spezifität für EGFR.

11. Das Her2-bindende Protein gemäß einem der vorhergehenden Ansprüche zur Verwendung in der Diagnostik oder Medizin, vorzugsweise zur Verwendung bei der Diagnose oder Behandlung von Krebs.

12. Nukleinsäuremolekül, welches für ein Her2-bindendes Protein kodiert, wie es in einem der Ansprüche 1 bis 11 definiert ist.

13. Vektor, umfassend das Nukleinsäuremolekül nach Anspruch 12.

14. Eine Wirtszelle oder ein nicht-menschlicher Wirt, umfassend das Her2-bindende Protein wie es in einem der Ansprüche 1 bis 11 definiert ist, eine Nukleinsäure nach Anspruch 12, und/oder einen Vektor nach Anspruch 13.

15. Zusammensetzung, umfassend das Her2-bindende Protein wie es in einem der Ansprüche 1 bis 11 definiert ist, ein Nukleinsäuremolekül nach Anspruch 12; den Vektor nach Anspruch 13; und/oder die Wirtszelle nach Anspruch 14.

16. Verfahren zur Herstellung eines Her2-bindenden Proteins nach einem der Ansprüche 1 bis 11, umfassend das Züchten der Wirtszelle nach Anspruch 14 unter geeigneten Bedingungen, um das Her2-bindende Protein zu erhalten, und gegebenenfalls Isolieren des Her2-bindenden Proteins.

## Revendications

1. Protéine de liaison à Her2 comprenant une séquence d'acides aminés dans laquelle 12, 13 ou 14 acides aminés choisis parmi des positions R42, I44, H68, V70, R72, L73, R74, K82, L84, Q138, K139, E140, S141 et T142 de la di-ubiquitine ayant la séquence d'acides aminés SEQ ID NO : 4 sont substitués, et dans laquelle la protéine de liaison à Her2 a au moins 85 % d'identité de séquence avec la di-ubiquitine de SEQ ID NO : 4, et dans laquelle la protéine de liaison à Her2 a une affinité de liaison (K_{D}) inférieure à 700 nM pour Her2, dans laquelle l'acide aminé à
une position 42 est substitué par un acide aminé polaire,
une position 44 est substitué par un acide aminé hydrophobe ou polaire,
une position 68 est substitué par un acide aminé aromatique,
une position 70 est substitué par un acide aminé aromatique,
une position 72 est substitué par un acide aminé polaire ou aromatique,
une position 73 est substitué par tout acide aminé mais pas un acide aminé basique ou acide,
une position 74 est substitué par un acide aminé aromatique, basique ou polaire,
une position 82 est substitué par tout acide aminé mais pas un acide aminé basique ou acide,
une position 84 est substitué par un acide aminé basique ou acide,
une position 138 est substitué par un acide aminé basique ou acide ou polaire,
une position 139 est substitué par un acide aminé acide ou hydrophobe ou une glycine,
une position 140 est substitué par un acide aminé aromatique,
une position 141 est substitué par un acide aminé hydrophobe ou polaire ou basique, et/ou
une position 142 est substitué par un acide aminé hydrophobe ou polaire.

2. Protéine de liaison à Her2 selon la revendication 1, comprenant 1, 2, 3, 4, 5 ou 6 autres substitutions de di-ubiquitine.

3. Protéine de liaison à Her2 selon la revendication 1, dans laquelle les acides aminés sont choisis parmi
R42T, R42S, R42L,
I44A, I44V, I44S, I44T,
H68W, H68Y, H68F,
V70Y, V7OW,
R72T, R72F, R72G, R72Y,
L73W, L73S, L73V, L73I,
R74Y, R74S, R74N, R74K,
K82T, K82L, K82N, K82I, K82Y,
L84H, L84D, L84E, L84S,
Q138S, Q138R, Q138E,
K139E, K139G, K139L,
E140W,
S141A,
S141R,
T142I, T142L, et/ou T142N.

4. Protéine de liaison à Her2 selon la revendication 3, dans laquelle les acides aminés sont choisis parmi
Q138S, K139E, E140W, S141A, et T142I ; ou
Q138R, K139G, E140W, et T142L ; ou
Q138E, K139L, E140W, S141R, et T142N.

5. Protéine de liaison à Her2 selon la revendication 3, dans laquelle les acides aminés sont choisis parmi
R42T, I44A, H68W, V70Y, R72T, L73W, R74Y, K82T, et L84H.

6. Protéine de liaison à Her2 selon la revendication 3, dans laquelle les acides aminés sont choisis parmi
R42S, I44V, H68Y, V70Y, R72F, L73S, K82L, et L84D.

7. Protéine de liaison à Her2 selon l'une quelconque des revendications précédentes, dans laquelle la séquence d'acides aminés est choisie parmi l'une des séquences d'acides aminés SEQ ID NO : 5 à 38.

8. Protéine de liaison à Her2 selon la revendication 3, dans laquelle la protéine de liaison à Her2 se lie à un épitope de Her2 différent ou ne se chevauchant pas par rapport à l'anticorps monoclonal Trastuzumab.

9. Protéine de liaison à Her2 selon l'une quelconque des revendications précédentes, comprenant en outre au moins une molécule supplémentaire, de préférence choisie parmi au moins un membre des groupes (i), (ii) et (iii) consistant en
(i) un fragment modulant une pharmacocinétique choisi parmi un polyéthylène glycol, une sérumalbumine humaine, un peptide de liaison à l'albumine, ou une immunoglobuline ou des fragments d'immunoglobuline, un polysaccharide, et
(ii) un composant thérapeutiquement actif, facultativement choisi parmi un anticorps monoclonal ou un fragment de celui-ci, une cytokine, une chimiokine, un composé cytotoxique, une enzyme, ou leurs dérivés, ou un radionucléide, et
(iii) un composant de diagnostic, facultativement choisi parmi un composé fluorescent, un photosensibilisateur, un marqueur, une enzyme ou, un radionucléide.

10. Protéine de liaison à Her2 selon la revendication 9, comprenant un anticorps monoclonal ayant une spécificité pour EGFR.

11. Protéine de liaison à Her2 selon l'une quelconque des revendications précédentes, destinée à être utilisée dans des diagnostics ou médicaments, de préférence destinée à être utilisée dans le diagnostic ou le traitement d'un cancer.

12. Molécule d'acide nucléique codant pour une protéine de liaison à Her2 telle que définie dans l'une quelconque des revendications 1 à 11.

13. Vecteur comprenant la molécule d'acide nucléique de la revendication 12.

14. Cellule hôte ou hôte non humain comprenant la protéine de liaison à Her2 telle que définie dans l'une quelconque des revendications 1 à 11, un acide nucléique tel que défini dans la revendication 12, et/ou un vecteur de la revendication 13.

15. Composition comprenant la protéine de liaison à Her2 telle que définie dans l'une quelconque des revendications 1 à 11, une molécule d'acide nucléique telle que définie dans la revendication 12 ; le vecteur tel que défini dans la revendication 13 ; et/ou la cellule hôte telle que définie dans la revendication 14.

16. Procédé de production d'une protéine de liaison à Her2 selon l'une quelconque des revendications 1 à 11, comprenant la culture de la cellule hôte de la revendication 14 dans des conditions appropriées afin d'obtenir ladite protéine de liaison à Her2, et facultativement l'isolement de ladite protéine de liaison à Her2.


### SEQUENCE LISTING

<110> Sci1 Proteins GmbH
<120> Her2 binding proteins based on di-ubiquitin muteins
<130> SP27 WO
<160> 50
<170> PatentIn version 3.5
<210> 1
   <211> 76
   <212> PRT
   <213> artificial
<220>
   <223> Ubiquitin (wildtype)
<400> 1
<210> 2
   <211> 76
   <212> PRT
   <213> artificial
<220>
   <223> Ubiquitin reference
<400> 2
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Trp Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Ala Ala
   65                  70                  75
<210> 3
   <211> 152
   <212> PRT
   <213> artificial
<220>
   <223> Ubiquitin reference
<220>
   <221> misc_feature
   <222> (42)..(42)
   <223> Xaa can be any naturally occurring amino acid
<220>
   <221> misc_feature
   <222> (44)..(44)
   <223> Xaa can be any naturally occurring amino acid
<220>
   <221> misc_feature
   <222> (68)..(68)
   <223> Xaa can be any naturally occurring amino acid
<220>
   <221> misc_feature
   <222> (70)..(70)
   <223> Xaa can be any naturally occurring amino acid
<220>
   <221> misc_feature
   <222> (72)..(74)
   <223> Xaa can be any naturally occurring amino acid
<220>
   <221> misc_feature
   <222> (82)..(82)
   <223> Xaa can be any naturally occurring amino acid
<220>
   <221> misc_feature
   <222> (84)..(84)
   <223> Xaa can be any naturally occurring amino acid
<220>
   <221> misc_feature
   <222> (138)..(142)
   <223> Xaa can be any naturally occurring amino acid
<400> 3
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Xaa Leu Xaa Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Xaa Leu Xaa Leu Xaa Xaa Xaa Ala Ala Met Gln Ile Phe
   65                  70                  75                  80
   Val Xaa Thr Xaa Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Xaa Xaa Xaa Xaa Xaa Leu His
       130                 135                 140
   Leu Val Leu Arg Leu Arg Ala Ala
   145                 150
<210> 4
   <211> 152
   <212> PRT
   <213> artificial
<220>
   <223> Ubiquitin reference (di-ubiquitin) clone 139090
<400> 4
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Ala Ala Met Gln Ile Phe
   65                  70                  75                  80
   Val Lys Thr Leu Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Gln Lys Glu Ser Thr Leu His
       130                 135                 140
   Leu Val Leu Arg Leu Arg Ala Ala
   145                 150
<210> 5
   <211> 152
   <212> PRT
   <213> artificial
<220>
   <223> Affilin-142437
<400> 5
   Met Gln Ile Phe Val Lys Thr Leu Thr Gly Lys Thr Ile Thr Leu Glu
   1               5                   10                  15
   Val Glu Pro Ser Asn Thr Ile Glu Asn Val Lys Ala Lys Ile Gln Asp
               20                  25                  30
   Lys Glu Gly Thr Pro Pro Asp Gln Gln Thr Leu Ala Phe Ala Gly Lys
           35                  40                  45
   His Leu Glu Asp Gly Arg Thr Leu Ser Asp Tyr Asn Ile Gln Lys Glu
       50                  55                  60
   Ser Thr Leu Trp Leu Tyr Leu Thr Trp Asn Ala Ala Met Arg Ile Phe
   65                  70                  75                  80
   Val Thr Thr His Thr Gly Lys Asn Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 6
   <211> 152
   <212> PRT
   <213> artificial
<220>
   <223> Affilin-142672
<400> 6
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Thr Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Leu Thr Asp Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Arg Gly Trp Ser Leu Leu His
       130                 135                 140
   Leu Val Leu Arg Leu Arg Ala Ala
   145                 150
<210> 7
   <211> 152
   <212> PRT
   <213> artificial
<220>
   <223> Affilin-144637
<400> 7
   Met Gln Ile Phe Val Lys Thr Leu Thr Gly Lys Thr Ile Thr Leu Glu
   1               5                   10                  15
   Val Glu Pro Ser Asp Thr Thr Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Tyr Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Asp Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 8
   <211> 152
   <212> PRT
   <213> artificial
<220>
   <223> Affilin-144636
<400> 8
   Met Gln Ile Phe Val Lys Thr Leu Thr Gly Lys Thr Ile Thr Leu Glu
   1               5                   10                  15
   Val Glu Pro Ser Asp Thr Thr Glu Asn Val Lys Ala Lys Ile Gln Asp
   20 25 30
   Lys Glu Gly Ile Pro Pro Asp Gln Gln Thr Leu Ala Phe Val Gly Lys
   35 40 45
   Gln Leu Glu Tyr Gly Arg Thr Leu Ser Asp Tyr Asn Ile Gln Lys Glu
   50 55 60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Ser Glu Trp Ala Ile Leu His
   130 135 140
   Leu Val Leu Arg Leu Arg Ala Ala
   145 150
<210> 9
   <211> 152
   <212> PRT
   <213> artificial
<220>
   <223> Affilin-144635
<400> 9
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Val Gly Met
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Asp Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Met Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 10
   <211> 152
   <212> PRT
   <213> artificial
<220>
   <223> Affilin-144634
<400> 10
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Asp Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Met Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 11
   <211> 152
   <212> PRT
   <213> artificial
<220>
   <223> Affilin-144633
<400> 11
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Tyr Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Asp Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 12
   <211> 152
   <212> PRT
   <213> artificial
<220>
   <223> Affilin-144632
<400> 12
   Met Gln Ile Phe Val Lys Thr Leu Thr Gly Lys Thr Ile Thr Leu Glu
   1               5                   10                  15
   Val Glu Pro Ser Asp Thr Thr Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 13
   <211> 152
   <212> PRT
   <213> artificial
<220>
   <223> Affilin-144631
<400> 13
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Val Gly Lys
   35 40 45
   Gln Leu Glu Asp Gly Arg Thr Leu Ser Asp Tyr Asn Ile Gln Lys Glu
   50 55 60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Asp Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Ser Glu Trp Ala Ile Leu His
   130 135 140
   Leu Val Leu Arg Leu Arg Ala Ala
   145 150
<210> 14
   <211> 152
   <212> PRT
   <213> artificial
<220>
   <223> Affilin-142679
<400> 14
   Met Gln Ile Phe Val Arg Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Thr Leu Ala Phe Val Gly Lys
   35 40 45
   Gln Leu Glu Asp Gly Arg Thr Leu Ser Asp Tyr Asn Ile Gln Lys Glu
   50 55 60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 15
   <211> 152
   <212> PRT
   <213> artificial
<220>
   <223> Affilin-142658
<400> 15
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Thr Leu Ala Phe Thr Gly Lys
   35 40 45
   Gln Leu Glu Asp Gly Arg Thr Leu Ser Asp Tyr Asn Ile Gln Lys Glu
   50 55 60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Ser Glu Trp Ala Ile Leu Gln
   130 135 140
   Leu Val Leu Arg Leu Arg Ala Ala
   145 150
<210> 16
   <211> 152
   <212> PRT
   <213> artificial
<220>
   <223> Affilin-142654
<400> 16
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Thr Leu Ala Phe Val Gly Lys
   35 40 45
   Gln Leu Glu Asp Gly Arg Thr Leu Ser Asp Tyr Asn Ile Gln Lys Glu
   50 55 60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Ser Glu Trp Ala Ile Leu His
   130 135 140
   Leu Val Leu Arg Leu Arg Ala Ala
   145 150
<210> 17
   <211> 152
   <212> PRT
   <213> artificial
<220>
   <223> Affilin-142653
<400> 17
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Ser Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 18
   <211> 152
   <212> PRT
   <213> artificial
<220>
   <223> Affilin-142645
<400> 18
   Met Gln Ile Phe Val Lys Thr Leu Thr Asp Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Ala Gly Lys
               35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Thr Asp Tyr Asn Ile Ser Glu Trp Ala Ile Leu His
       130                 135                 140
   Leu Val Leu Arg Leu Arg Ala Ala
   145                 150
<210> 19
   <211> 152
   <212> PRT
   <213> artificial
<220>
   <223> Affilin-142628
<400> 19
   Met Gln Ile Phe Val Lys Thr Leu Thr Gly Lys Thr Ile Thr Leu Glu
   1               5                   10                  15
   Val Glu Pro Ser Asp Thr Thr Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Asp Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 20
   <211> 152
   <212> PRT
   <213> artificial
<220>
   <223> Affilin-142620
<400> 20
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Val Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 21
   <211> 152
   <212> PRT
   <213> artificial
<220>
   <223> Affilin-142618
<400> 21
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Lys Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Gln Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 22
   <211> 152
   <212> PRT
   <213> artificial
<220>
   <223> Affilin-142617
<400> 22
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Thr Leu Ala Phe Val Gly Lys
   35 40 45
   Gln Leu Glu Asp Gly Arg Thr Leu Ser Asp Tyr Asn Ile Gln Lys Glu
   50 55 60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Ser Asp Gln Gln Arg Leu Ile Trp Ala Gly Lys Gln Leu Glu Asp
   115 120 125
   Gly Arg Ala Leu Ser Asp Tyr Asn Ile Ser Glu Trp Ala Ile Leu His
   130 135 140
   Leu Val Leu Arg Leu Arg Ala Ala
   145 150
<210> 23
   <211> 152
   <212> PRT
   <213> artificial
<220>
   <223> Affilin-142609
<400> 23
   Met Gln Ile Phe Val Lys Thr Leu Thr Gly Asn Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Thr Leu Ala Phe Val Gly Lys
   35 40 45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Pro Arg Leu Arg Ala Ala
   145                 150
<210> 24
   <211> 152
   <212> PRT
   <213> artificial
<220>
   <223> Affilin-142451
<400> 24
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Thr
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Asn Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 25
   <211> 152
   <212> PRT
   <213> artificial
<220>
   <223> Affilin-142441
<400> 25
   Met Gln Ile Phe Val Lys Thr Leu Thr Asn Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
                       145                 150
<210> 26
   <211> 152
   <212> PRT
   <213> artificial
<220>
   <223> Affilin-142439
<400> 26
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Ala Gly Lys
           35                  40                  45
   Gln Leu Glu Tyr Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 27
   <211> 152
   <212> PRT
   <213> artificial
<220>
   <223> Affilin-141931
<400> 27
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Val Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Gln Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 28
   <211> 152
   <212> PRT
   <213> artificial
<220>
   <223> Affilin-141926
<400> 28
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Thr Leu Ala Phe Ala Gly Lys
   35 40 45
   Gln Leu Glu Asp Gly Arg Thr Leu Ser Asp Tyr Asn Ile Gln Lys Glu
   50 55 60
   Ser Thr Leu Trp Leu Tyr Leu Thr Trp Tyr Ala Ala Met Arg Ile Phe
   65 70 75 80
   Val Thr Thr His Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Ser Glu Trp Ala Ile Leu His
   130 135 140
   Leu Val Leu Arg Leu Arg Ala Ala
   145 150
<210> 29
   <211> 152
   <212> PRT
   <213> artificial
<220>
   <223> Affilin-141869
<400> 29
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Gln Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 30
   <211> 152
   <212> PRT
   <213> artificial
<220>
   <223> Affilin-141890
<400> 30
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Thr Leu Ala Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Gln Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 31
   <211> 152
   <212> PRT
   <213> artificial
<220>
   <223> Affilin-141912
<400> 31
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Val Pro Pro Asp Gln Gln Thr Leu Ile Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Phe Leu Tyr Leu Tyr Val Ser Ala Ala Met Gln Ile Phe
   65                  70                  75                  80
   Val Leu Thr Asp Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Glu Leu Trp Arg Asn Leu His
       130                 135                 140
   Leu Val Leu Arg Leu Arg Ala Ala
   145                 150
<210> 32
   <211> 152
   <212> PRT
   <213> artificial
<220>
   <223> Affilin-141935
<400> 32
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
   20 25 30
   Lys Asp Gly Ile Pro Pro Asp Gln Gln Thr Leu Thr Phe Ala Gly Lys
   35 40 45
   Gln Leu Glu Asp Gly Arg Thr Leu Ser Asp Tyr Asn Ile Gln Lys Glu
   50 55 60
   Ser Thr Leu Tyr Leu Tyr Leu Gly Ile Ser Ala Ala Met Gln Ile Phe
   65 70 75 80
   Val Ile Thr Ser Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Glu Leu Trp Arg Asn Leu His
   130 135 140
   Leu Val Leu Arg Leu Arg Ala Ala
   145 150
<210> 33
   <211> 152
   <212> PRT
   <213> artificial
<220>
   <223> Affilin-142465
<400> 33
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Leu Leu Ser Phe Ala Gly Lys
   35 40 45
   Gln Leu Glu Asp Gly Arg Thr Leu Ser Asp Tyr Asn Ile Gln Lys Glu
   50 55 60
   Ser Thr Leu His Leu Trp Leu Gly Val Arg Ala Ala Met Gln Ile Phe
   65                  70                  75                  80
   Val Asn Thr Glu Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 34
   <211> 152
   <212> PRT
   <213> artificial
<220>
   <223> Affilin-142655
<400> 34
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Leu Asp
               20                  25                  30
   Lys Glu Gly Ile Pro Pro Asp Gln Gln Ser Leu Val Phe Ala Gly Lys
           35                  40                  45
   Gln Leu Glu Asp Gly Arg Thr Leu Ser Val Tyr Asn Ile Gln Lys Glu
       50                  55                  60
   Ser Thr Leu Tyr Leu Tyr Leu Phe Ser Arg Ala Ala Met Arg Ile Phe
   65                  70                  75                  80
   Val Leu Thr Asp Thr Gly Lys Thr Ile Thr Leu Glu Val Glu Ser Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Arg Gly Trp Ser Leu Leu His
   130 135 140
   Leu Val Leu Arg Leu Arg Ala Ala
   145 150
<210> 35
   <211> 152
   <212> PRT
   <213> artificial
<220>
   <223> Affilin-142418
<400> 35
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Ser Leu Val Phe Ala Gly Lys
   35 40 45
   Gln Leu Glu Asp Gly Arg Thr Leu Ser Asp Tyr Asn Ile Gln Ile Glu
   50 55 60
   Ser Thr Leu Tyr Leu Tyr Leu Phe Ser Arg Ala Ala Met Arg Ile Phe
   65 70 75 80
   Val Leu Thr Asp Thr Gly Lys Thr Ile Thr Leu Glu Val Glu Pro Ser
   85 90 95
   Asp Thr Ile Glu Asn Val Lys Ala Lys Ile Gln Asp Lys Glu Gly Ile
   100 105 110
   Pro Pro Asp Gln Gln Arg Leu Ile Trp Ala Gly Lys Gln Leu Glu Asp
   115 120 125
   Gly Arg Thr Leu Ser Asp Tyr Asn Ile Arg Gly Trp Ser Leu Leu His
   130 135 140
   Leu Val Leu Arg Leu Arg Ala Ala
   145 150
<210> 36
   <211> 152
   <212> **PRT**
   <213> artificial
<220>
   <223> Affilin-142627
<400> 36
   Met Gln Ile Phe Val Lys Thr Leu Thr Gly Lys Thr Ile Thr Val Glu
   1               5                   10                  15
   Val Glu Pro Cys Asp Thr Ile Glu Asn Val Lys Ala Lys Ile Gln Asp
               20                  25                  30
   Lys Glu Gly Ile Pro Pro Asp Gln Gln Ser Leu Val Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Tyr Leu Tyr Leu Phe Ser Arg Ala Ala Met Arg Ile Phe
   65                  70                  75                  80
   Val Leu Thr Asp Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 37
   <211> 152
   <212> PRT
   <213> artificial
<220>
   <223> Affilin-141975
<400> 37
   Met Gln Ile Phe Val Lys Thr Leu Thr Gly Lys Thr Ile Thr Leu Glu
   1 5 10 15
   Val Glu Pro Ser Asp Thr Ile Glu Asn Val Lys Ala Lys Ile Gln Asp
   20 25 30
   Lys Glu Gly Ile Pro Pro Asp Gln Gln Ser Leu Val Phe Ala Gly Lys
   35 40 45
   Gln Leu Glu Tyr Gly Arg Thr Leu Ser Asp Tyr Asn Ile Gln Lys Glu
   50 55 60
   Ser Thr Leu Tyr Leu Tyr Leu Phe Ser Arg Val Ala Met Arg Ile Phe
   65 70 75 80
   Val Leu Thr Asp Thr Gly Lys Thr Ile Thr Leu Glu Val Glu Pro Asn
   85 90 95
   Asp Thr Ile Glu Asn Val Lys Ala Lys Ile Gln Asp Lys Glu Gly Ile
   100 105 110
   Pro Ser Asp Gln Gln Arg Leu Ile Trp Ala Gly Lys Gln Leu Glu Asp
   115 120 125
   Gly Arg Ala Leu Ser Asp Tyr Asn Ile Ser Glu Trp Ala Ile Leu His
   130 135 140
   Leu Val Leu Arg Leu Arg Ala Ala
   145 150
<210> 38
   <211> 152
   <212> PRT
   <213> artificial
<220>
   <223> Affilin-141884
<400> 38
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Arg Ala Lys Ile Gln Asp
               20                  25                  30
   Lys Glu Gly Ile Pro Pro Asp Gln Gln Thr Leu Ala Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Trp Leu Tyr Leu Thr Trp Tyr Ala Ala Met Arg Ile Phe
   65                  70                  75                  80
   Val Thr Thr His Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Ser Glu Trp Ala Ile Leu His
       130                 135                 140
   Leu Val Leu Arg Leu Arg Ala Ala
   145                 150
<210> 39
   <211> 82
   <212> PRT
   <213> artificial
<220>
   <223> LOOP BINDER 1>144567 (6er LOOP)
<400> 39
   Met Gln Ile Phe Val Lys Thr Leu Thr Pro Tyr Glu Thr Gln Val Gly
   1               5                   10                  15
   Lys Thr Ile Thr Leu Glu Val Glu Pro Ser Asp Thr Ile Glu Asn Val
               20                  25                  30
   Lys Ala Lys Ile Gln Asp Lys Glu Gly Ile Pro Pro Asp Gln Gln Arg
           35                  40                  45
   Leu Ile Trp Ala Gly Lys Gln Leu Glu Asp Gly Arg Thr Leu Ser Asp
       50                  55                  60
   Tyr Asn Ile Arg Gly Gly Arg Phe Leu Trp Leu Lys Leu Lys Leu Arg
   65                  70                  75                  80
   Ala Ala
<210> 40
   <211> 85
   <212> PRT
   <213> artificial
<220>
   <223> LOOP BINDER 2>143692 (9er LOOP)
<400> 40
   Met Gln Ile Phe Val Lys Thr Leu Thr Ala Gly Asn Pro Ser His Met
   1               5                   10                  15
   His His Gly Lys Thr Ile Thr Leu Glu Val Glu Pro Ser Asp Thr Ile
               20                  25                  30
   Glu Asn Val Lys Ala Lys Ile Gln Asp Lys Glu Gly Ile Pro Pro Asp
           35                  40                  45
   Gln Gln Arg Leu Ile Trp Ala Gly Lys Gln Leu Glu Asp Gly Arg Thr
       50                  55                  60
   Leu Ser Asp Tyr Asn Ile Asp Asp Met His Trp Leu Ile Leu Leu Leu
   65                  70                  75                  80
   Asn Leu Arg Ala Ala
                   85
<210> 41
   <211> 4
   <212> PRT
   <213> artificial
<220>
   <223> General linker sequence
<400> 41
   Ser Gly Gly Gly
   1
<210> 42
   <211> 6
   <212> PRT
   <213> artificial
<220>
   <223> Insertion of 6 amino acid in Affilin-144567
<400> 42
   Pro Tyr Glu Thr Gln Val
   1 5
<210> 43
   <211> 9
   <212> PRT
   <213> artificial
<220>
   <223> Insertion of 9 amino acids in Affilin-143692
<400> 43
   Ala Gly Asn Pro Ser His Met His His
   1 5
<210> 44
   <211> 401
   <212> PRT
   <213> artificial
<220>
   <223> Fusion protein of Affilin SEQ ID NO: 28 to c-terminus of light chain of Cetuximab (refered to as CL-141926)
<400> 44
   Met Val Ser Thr Pro Gln Phe Leu Val Phe Leu Leu Phe Trp Ile Pro
   1               5                   10                  15
   Ala Ser Arg Ser Asp Ile Leu Leu Thr Gln Ser Pro Val Ile Leu Ser
               20                  25                  30
   Val Ser Pro Gly Glu Arg Val Ser Phe Ser Cys Arg Ala Ser Gln Ser
           35                  40                  45
   Ile Gly Thr Asn Ile His Trp Tyr Gln Gln Arg Thr Asn Gly Ser Pro
       50                  55                  60
   Arg Leu Leu Ile Lys Tyr Ala Ser Glu Ser Ile Ser Gly Ile Pro Ser
   65                  70                  75                  80
   Arg Phe Ser Gly Ser Gly Ser Gly Thr Asp Phe Thr Leu Ser Ile Asn
                   85                  90                  95
   Ser Val Glu Ser Glu Asp Ile Ala Asp Tyr Tyr Cys Gln Gln Asn Asn
               100                 105                 110
   Asn Trp Pro Thr Thr Phe Gly Ala Gly Thr Lys Leu Glu Leu Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys Gly Gly Gly Gly Ser Gly
   225                 230                 235                 240
   Gly Gly Gly Ser Gly Gly Gly Gly Ser Met Gln Ile Phe Val Lys Thr
                   245                 250                 255
   Leu Thr Gly Lys Thr Ile Thr Leu Glu Val Glu Pro Ser Asp Thr Ile
               260                 265                 270
   Glu Asn Val Lys Ala Lys Ile Gln Asp Lys Glu Gly Ile Pro Pro Asp
           275                 280                 285
   Gln Gln Thr Leu Ala Phe Ala Gly Lys Gln Leu Glu Asp Gly Arg Thr
       290                 295                 300
   Leu Ser Asp Tyr Asn Ile Gln Lys Glu Ser Thr Leu Trp Leu Tyr Leu
   305                 310                 315                 320
   Thr Trp Tyr Ala Ala Met Arg Ile Phe Val Thr Thr His Thr Gly Lys
                   325                 330                 335
   Thr Ile Thr Leu Glu Val Glu Pro Ser Asp Thr Ile Glu Asn Val Lys
               340                 345                 350
   Ala Lys Ile Gln Asp Lys Glu Gly Ile Pro Pro Asp Gln Gln Arg Leu
           355                 360                 365
   Ile Trp Ala Gly Lys Gln Leu Glu Asp Gly Arg Thr Leu Ser Asp Tyr
       370                 375                 380
   Asn Ile Ser Glu Trp Ala Ile Leu His Leu Val Leu Arg Leu Arg Ala
   385                 390                 395                 400
   Ala
<210> 45
   <211> 635
   <212> PRT
   <213> artificial
<220>
   <223> Fusion protein of Affilin SEQ ID NO: 28 to c-terminus of heavy chain of Cetuximab (refered to as CH-141926)
<400> 45
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Glu Glu Met Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Lys Leu Thr
               420                 425                 430
   Val Asp Lys Ser Arg Trp Gln Gln Gly Asn Val Phe Ser Cys Ser Val
           435                 440                 445
   Met His Glu Ala Leu His Asn His Tyr Thr Gln Lys Ser Leu Ser Leu
       450                 455                 460
   Ser Pro Gly Lys Gly Gly Gly Gly Ser Gly Gly Gly Gly Ser Gly Gly
   465                 470                 475                 480
   Gly Gly Ser Met Gln Ile Phe Val Lys Thr Leu Thr Gly Lys Thr Ile
   485 490 495
   Thr Leu Glu Val Glu Pro Ser Asp Thr Ile Glu Asn Val Lys Ala Lys
   500 505 510
   Ile Gln Asp Lys Glu Gly Ile Pro Pro Asp Gln Gln Thr Leu Ala Phe
   515 520 525
   Ala Gly Lys Gln Leu Glu Asp Gly Arg Thr Leu Ser Asp Tyr Asn Ile
   530 535 540
   Gln Lys Glu Ser Thr Leu Trp Leu Tyr Leu Thr Trp Tyr Ala Ala Met
   545 550 555 560
   Arg Ile Phe Val Thr Thr His Thr Gly Lys Thr Ile Thr Leu Glu Val
   565 570 575
   Glu Pro Ser Asp Thr Ile Glu Asn Val Lys Ala Lys Ile Gln Asp Lys
   580 585 590
   Glu Gly Ile Pro Pro Asp Gln Gln Arg Leu Ile Trp Ala Gly Lys Gln
   595 600 605
   Leu Glu Asp Gly Arg Thr Leu Ser Asp Tyr Asn Ile Ser Glu Trp Ala
   610 615 620
   Ile Leu His Leu Val Leu Arg Leu Arg Ala Ala
   625 630 635
<210> 46
   <211> 401
   <212> PRT
   <213> artificial
<220>
   <223> Fusion protein of Affilin SEQ ID NO: 28 to n-terminus of light chain of Cetuximab (refered to as NL-141926)
<400> 46
   Met Val Ser Thr Pro Gln Phe Leu Val Phe Leu Leu Phe Trp Ile Pro
   1 5 10 15
   Ala Ser Arg Ser Met Gln Ile Phe Val Lys Thr Leu Thr Gly Lys Thr
   20 25 30
   Ile Thr Leu Glu Val Glu Pro Ser Asp Thr Ile Glu Asn Val Lys Ala
   35 40 45
   Lys Ile Gln Asp Lys Glu Gly Ile Pro Pro Asp Gln Gln Thr Leu Ala
       50                  55                  60
   Phe Ala Gly Lys Gln Leu Glu Asp Gly Arg Thr Leu Ser Asp Tyr Asn
   65                  70                  75                  80
   Ile Gln Lys Glu Ser Thr Leu Trp Leu Tyr Leu Thr Trp Tyr Ala Ala
                   85                  90                  95
   Met Arg Ile Phe Val Thr Thr His Thr Gly Lys Thr Ile Thr Leu Glu
               100                 105                 110
   Val Glu Pro Ser Asp Thr Ile Glu Asn Val Lys Ala Lys Ile Gln Asp
           115                 120                 125
   Lys Glu Gly Ile Pro Pro Asp Gln Gln Arg Leu Ile Trp Ala Gly Lys
       130                 135                 140
   Gln Leu Glu Asp Gly Arg Thr Leu Ser Asp Tyr Asn Ile Ser Glu Trp
   145                 150                 155                 160
   Ala Ile Leu His Leu Val Leu Arg Leu Arg Ala Ala Gly Gly Gly Gly
                   165                 170                 175
   Ser Gly Gly Gly Gly Ser Gly Gly Gly Gly Ser Asp Ile Leu Leu Thr
               180                 185                 190
   Gln Ser Pro Val Ile Leu Ser Val Ser Pro Gly Glu Arg Val Ser Phe
           195                 200                 205
   Ser Cys Arg Ala Ser Gln Ser Ile Gly Thr Asn Ile His Trp Tyr Gln
       210                 215                 220
   Gln Arg Thr Asn Gly Ser Pro Arg Leu Leu Ile Lys Tyr Ala Ser Glu
   225                 230                 235                 240
   Ser Ile Ser Gly Ile Pro Ser Arg Phe Ser Gly Ser Gly Ser Gly Thr
                   245                 250                 255
   Asp Phe Thr Leu Ser Ile Asn Ser Val Glu Ser Glu Asp Ile Ala Asp
               260                 265                 270
   Tyr Tyr Cys Gln Gln Asn Asn Asn Trp Pro Thr Thr Phe Gly Ala Gly
           275                 280                 285
   Thr Lys Leu Glu Leu Lys Arg Thr Val Ala Ala Pro Ser Val Phe Ile
       290                 295                 300
   Phe Pro Pro Ser Asp Glu Gln Leu Lys Ser Gly Thr Ala Ser Val Val
   305                 310                 315                 320
   Cys Leu Leu Asn Asn Phe Tyr Pro Arg Glu Ala Lys Val Gln Trp Lys
                   325                 330                 335
   Val Asp Asn Ala Leu Gln Ser Gly Asn Ser Gln Glu Ser Val Thr Glu
               340                 345                 350
   Gln Asp Ser Lys Asp Ser Thr Tyr Ser Leu Ser Ser Thr Leu Thr Leu
           355                 360                 365
   Ser Lys Ala Asp Tyr Glu Lys His Lys Val Tyr Ala Cys Glu Val Thr
       370                 375                 380
   His Gln Gly Leu Ser Ser Pro Val Thr Lys Ser Phe Asn Arg Gly Glu
   385                 390                 395                 400
   Cys
<210> 47
   <211> 643
   <212> PRT
   <213> artificial
<220>
   <223> Fusion protein of Affilin SEQ ID NO: 28 to n-terminus of heavy chain of Cetuximab (refered to as NH-141926)
<400> 47
   Met Ala Val Leu Gly Leu Leu Phe Cys Leu Val Thr Phe Pro Ser Cys
   1               5                   10                  15
   Val Leu Ser Met Gln Ile Phe Val Lys Thr Leu Thr Gly Lys Thr Ile
               20                  25                  30
   Thr Leu Glu Val Glu Pro Ser Asp Thr Ile Glu Asn Val Lys Ala Lys
           35                  40                  45
   Ile Gln Asp Lys Glu Gly Ile Pro Pro Asp Gln Gln Thr Leu Ala Phe
       50                  55                  60
   Ala Gly Lys Gln Leu Glu Asp Gly Arg Thr Leu Ser Asp Tyr Asn Ile
   65                  70                  75                  80
   Gln Lys Glu Ser Thr Leu Trp Leu Tyr Leu Thr Trp Tyr Ala Ala Met
                   85                  90                  95
   Arg Ile Phe Val Thr Thr His Thr Gly Lys Thr Ile Thr Leu Glu Val
   100 105 110
   Glu Pro Ser Asp Thr Ile Glu Asn Val Lys Ala Lys Ile Gln Asp Lys
   115 120 125
   Glu Gly Ile Pro Pro Asp Gln Gln Arg Leu Ile Trp Ala Gly Lys Gln
   130 135 140
   Leu Glu Asp Gly Arg Thr Leu Ser Asp Tyr Asn Ile Ser Glu Trp Ala
   145 150 155 160
   Ile Leu His Leu Val Leu Arg Leu Arg Ala Ala Gly Gly Gly Gly Ser
   165 170 175
   Gly Gly Gly Gly Ser Gly Gly Gly Gly Ser Gln Val Gln Leu Lys Gln
   180 185 190
   Ser Gly Pro Gly Leu Val Gln Pro Ser Gln Ser Leu Ser Ile Thr Cys
   195 200 205
   Thr Val Ser Gly Phe Ser Leu Thr Asn Tyr Gly Val His Trp Val Arg
   210 215 220
   Gln Ser Pro Gly Lys Gly Leu Glu Trp Leu Gly Val Ile Trp Ser Gly
   225 230 235 240
   Gly Asn Thr Asp Tyr Asn Thr Pro Phe Thr Ser Arg Leu Ser Ile Asn
   245 250 255
   Lys Asp Asn Ser Lys Ser Gln Val Phe Phe Lys Met Asn Ser Leu Gln
   260 265 270
   Ser Asn Asp Thr Ala Ile Tyr Tyr Cys Ala Arg Ala Leu Thr Tyr Tyr
   275 280 285
   Asp Tyr Glu Phe Ala Tyr Trp Gly Gln Gly Thr Leu Val Thr Val Ser
   290 295 300
   Ala Ala Ser Thr Lys Gly Pro Ser Val Phe Pro Leu Ala Pro Ser Ser
   305 310 315 320
   Lys Ser Thr Ser Gly Gly Thr Ala Ala Leu Gly Cys Leu Val Lys Asp
   325 330 335
   Tyr Phe Pro Glu Pro Val Thr Val Ser Trp Asn Ser Gly Ala Leu Thr
               340                 345                 350
   Ser Gly Val His Thr Phe Pro Ala Val Leu Gln Ser Ser Gly Leu Tyr
           355                 360                 365
   Ser Leu Ser Ser Val Val Thr Val Pro Ser Ser Ser Leu Gly Thr Gln
       370                 375                 380
   Thr Tyr Ile Cys Asn Val Asn His Lys Pro Ser Asn Thr Lys Val Asp
   385                 390                 395                 400
   Lys Arg Val Glu Pro Lys Ser Cys Asp Lys Thr His Thr Cys Pro Pro
                   405                 410                 415
   Cys Pro Ala Pro Glu Leu Leu Gly Gly Pro Ser Val Phe Leu Phe Pro
               420                 425                 430
   Pro Lys Pro Lys Asp Thr Leu Met Ile Ser Arg Thr Pro Glu Val Thr
           435                 440                 445
   Cys Val Val Val Asp Val Ser His Glu Asp Pro Glu Val Lys Phe Asn
       450                 455                 460
   Trp Tyr Val Asp Gly Val Glu Val His Asn Ala Lys Thr Lys Pro Arg
   465                 470                 475                 480
   Glu Glu Gln Tyr Asn Ser Thr Tyr Arg Val Val Ser Val Leu Thr Val
                   485                 490                 495
   Leu His Gln Asp Trp Leu Asn Gly Lys Glu Tyr Lys Cys Lys Val Ser
               500                 505                 510
   Asn Lys Ala Leu Pro Ala Pro Ile Glu Lys Thr Ile Ser Lys Ala Lys
           515                 520                 525
   Gly Gln Pro Arg Glu Pro Gln Val Tyr Thr Leu Pro Pro Ser Arg Glu
       530                 535                 540
   Glu Met Thr Lys Asn Gln Val Ser Leu Thr Cys Leu Val Lys Gly Phe
   545                 550                 555                 560
   Tyr Pro Ser Asp Ile Ala Val Glu Trp Glu Ser Asn Gly Gln Pro Glu
                   565                 570                 575
   Asn Asn Tyr Lys Thr Thr Pro Pro Val Leu Asp Ser Asp Gly Ser Phe
               580                 585                 590
   Phe Leu Tyr Ser Lys Leu Thr Val Asp Lys Ser Arg Trp Gln Gln Gly
           595                 600                 605
   Asn Val Phe Ser Cys Ser Val Met His Glu Ala Leu His Asn His Tyr
       610                 615                 620
   Thr Gln Lys Ser Leu Ser Leu Ser Pro Gly Lys Trp Ser His Pro Gln
   625                 630                 635                 640
   Phe Glu Lys
<210> 48
   <211> 152
   <212> PRT
   <213> artificial
<220>
   <223> Ubiquitin reference (di-ubiquitin)
<400> 48
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Arg Leu Ile Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu His Leu Val Leu Arg Leu Arg Gly Gly Met Gln Ile Phe
   65                  70                  75                  80
   Val Lys Thr Leu Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Phe Ala Gly Lys Gln Leu Glu Asp
           115                 120                 125
   Gly Arg Thr Leu Ser Asp Tyr Asn Ile Gln Lys Glu Ser Thr Leu His
       130                 135                 140
   Leu Val Leu Arg Leu Arg Gly Gly
                       145                 150
<210> 49
   <211> 152
   <212> PRT
   <213> artificial
<220>
   <223> Clone 142502
<400> 49
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Ser Leu Val Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Tyr Leu Tyr Leu Phe Ser Arg Ala Ala Met Gln Ile Phe
   65                  70                  75                  80
   Val Leu Thr Asp Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Lys Gln Leu Glu Asp
           115                 120                 125
   Gly Arg Thr Leu Ser Asp Tyr Asn Ile Arg Gly Trp Ser Leu Leu His
       130                 135                 140
   Leu Val Leu Arg Leu Arg Ala Ala
   145                 150
<210> 50
   <211> 152
   <212> PRT
   <213> artificial
<220>
   <223> Clone 141965
<400> 50
   Met Gln Ile Phe Val Lys Thr Leu Thr Gly Lys Thr Ile Thr Leu Glu
   1               5                   10                  15
   Val Glu Pro Ser Asp Thr Ile Glu Asn Val Lys Ala Lys Ile Gln Asp
               20                  25                  30
   Lys Glu Gly Ile Pro Pro Asp Gln Gln Ser Leu Val Phe Ala Gly Lys
           35                  40                  45
   Gln Leu Glu Asp Gly Arg Thr Leu Ser Asp Tyr Asn Ile Gln Lys Glu
       50                  55                  60
   Ser Thr Leu Tyr Leu Tyr Leu Phe Ser Arg Ala Ala Met Gln Ile Phe
   65                  70                  75                  80
   Val Leu Thr Asp Thr Gly Lys Thr Ile Thr Leu Glu Val Glu Pro Ser
                   85                  90                  95
   Asp Thr Ile Glu Asn Val Lys Ala Lys Ile Gln Asp Lys Glu Gly Ile
               100                 105                 110
   Pro Pro Asp Gln Gln Arg Leu Ile Trp Ala Gly Met Gln Leu Glu Asp
           115                 120                 125
   Gly Arg Thr Leu Ser Asp Tyr Asn Ile Arg Gly Trp Ser Leu Leu His
       130                 135                 140
   Leu Val Leu Arg Leu Arg Ala Ala
   145                 150